(19) Europäisches Patentamt
European Patent Office
Office européen des brevets

(11) EP 4 212 084 A1

(12) EUROPEAN PATENT APPLICATION
published in accordance with Art. 153(4) EPC

(43) Date of publication:
19.07.2023 Bulletin 2023/29

(51) International Patent Classification (IPC):
A47L 11/24 (2006.01)  A47L 11/40 (2006.01)
A61L 2/10 (2006.01)  G01F 23/18 (2006.01)
G08B 21/18 (2006.01)  G05B 19/042 (2006.01)

(21) Application number: 21863575.3

(22) Date of filing: 30.08.2021

(52) Cooperative Patent Classification (CPC):
A47L 11/24; A47L 11/40; A61L 2/10; G01F 23/18;
G05B 19/042; G08B 21/18

(86) International application number:
PCT/CN2021/115247

(87) International publication number:
WO 2022/048510 (10.03.2022 Gazette 2022/10)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 07.09.2020  CN 202010929134
07.09.2020  CN 202010928370
07.09.2020  CN 202021928732 U
07.09.2020  CN 202010928363
07.09.2020  CN 202021928734 U

(71) Applicant: Dreame Innovation Technology
(Suzhou) Co., Ltd.
Suzhou, Jiangsu 215104 (CN)

(72) Inventors:
• WU, Yadong
Suzhou, Jiangsu 215104 (CN)
• FU, Haiyang
Suzhou, Jiangsu 215104 (CN)
• XIA, Lei
Suzhou, Jiangsu 215104 (CN)
• XU, Bojian
Suzhou, Jiangsu 215104 (CN)

(74) Representative: dompatent von Kreisler Selting
Werner -
Partnerschaft von Patent- und Rechtsanwälten
mbB
Deichmannhaus am Dom
Bahnhofsvorplatz 1
50667 Köln (DE)

(54) INTELLIGENT CLEANING SYSTEM

(57) The present application relates to an intelligent cleaning system, comprising a robot cleaner and a maintenance station fitted to the robot cleaner. The maintenance station comprises: a first housing provided with a dust extraction port and an air discharge port; a first fan disposed on the first housing, an air discharge end of the first fan being respectively in communication with the air discharge port and the dust extraction port; and a charging component disposed on the first housing for charging the robot cleaner. The robot cleaner comprises: a second housing provided with a dust suction port, a dust outlet, and an air blowing port, the dust outlet and the air blowing port being arranged on both opposite ends of the second housing; the dust outlet being fitted to the dust extraction port; the air blowing port being fitted to the air discharge port; and a working component disposed on the second housing, so that the robot cleaner moves and cleans. The intelligent cleaning system can effectively improve the clearance rate of a robot cleaner box to ensure the thorough cleaning of the interior of the robot cleaner box and avoid mildewing.

FIG. 1

## Description

## TECHNICAL FIELD

The present application relates to an intelligent cleaning system, which belongs to the field of intelligent household appliances.

## BACKGROUND

Generally, a sweeping robot dust extraction system includes a sweeping robot and a maintenance station. Its working principle is as follows: there is a fan in the maintenance station; when the sweeping robot finishes cleaning, it automatically returns to the maintenance station. Relying on the wind pressure generated by the rotation of the fan in the maintenance station, the garbage in a dust box of the sweeping robot is drawn away. Since the volume of the dust box of the maintenance station is much larger than that of the sweeping robot, the user does not need to clean the dust in the dust box of the sweeping robot, thereby reducing the frequency of user dumping.

However, there is only one dust discharge port on the sweeping robot of the existing sweeping robot dust extraction system. However, the size of the dust discharge port is usually much smaller than that of the dust box. The dust discharge port is arranged on one side of a bottom surface of the dust box. In this way, there are dead corners for cleaning in the dust box of the sweeping robot. The dust discharge rate (clearance rate) is not high. On the one hand, users still need to clean it from time to time; and on the other hand, the garbage that has existed in the dead corner for a long time has the risk of mildew.

Besides, when the existing cleaning machine dust extraction system performs cleaning tasks, the maintenance station only runs for a fixed time and then stops. If there are a lot of flocculent debris in the dust box or the dust suction channel is blocked, the dust in the dust box of the sweeping robot will not be completely emptied. However, the dust extraction work has ended at this time, but the fan of the maintenance station is still running, resulting in wasted energy and noise.

## SUMMARY

According to a first aspect, a purpose of the present application is to provide an intelligent cleaning system, which can effectively improve the clearance rate of the dust box of the sweeping robot, so as to ensure that an interior of the dust box of the sweeping robot is completely clean and prevent mildew.

To achieve the above purpose, the present application provides the following technical solution:
an intelligent cleaning system, including: a sweeping robot and a maintenance station configured to mate with the sweeping robot, the maintenance station including:

a first shell being provided with a dust extraction port and an air discharge port;
a first fan arranged on the first shell, an air discharge end and an air suction end of the first fan communicating with the air discharge port and the dust extraction port, respectively; and
a charging assembly disposed on the first shell and configured to charge the sweeping robot;
the sweeping robot including:

a second shell provided with a dust suction port, a dust outlet port and an air blowing port; the dust outlet port and the air blowing port are arranged at opposite ends of the second shell; the dust outlet port is configured to mate with the dust extraction port, and the air blowing port is configured to mate with the air discharge port; and
a working assembly arranged on the second shell to drive the sweeping robot to move and clean.

Further, the second shell has a bottom surface; the dust outlet port and the air flowing port are arranged at opposite ends of the bottom surface, respectively.

Further, the bottom surface includes a first bottom surface and a second bottom surface; a height of the second bottom surface is higher than that of the first bottom surface in a vertical direction of the sweeping robot; the dust outlet port and the air flowing port are arranged on the first bottom surface, and the dust suction port is arranged on the second bottom surface.

Further, an air inlet valve is provided at the air flowing port; when the sweeping robot is in a working state, the air inlet valve closes the air flowing port; when the sweeping robot is mated with the maintenance station, the air inlet valve opens the air flowing port, so that the air flowing port communicates with the air discharge port.

Further, a dust discharge valve is provided at the dust outlet port; when the sweeping robot is in the working state, the dust discharge valve closes the dust outlet port; when the sweeping robot is mated with the maintenance station, the dust discharge valve opens the dust outlet port, so that the dust outlet port communicates with the dust extraction port.

Further, the second shell has an accommodating space; and when the sweeping robot is in a working state, the accommodating space is in a negative pressure state.

Further, the working assembly includes a ground brush assembly disposed at the dust suction port, a driving assembly driving the ground brush assembly to rotate, a moving assembly for moving the sweeping robot, and a second fan for making the accommodating space in a negative pressure state.

Further, the working assembly further includes a filter which is arranged between the second fan and the accommodating space; when the sweeping robot is in the working state, dust-carrying airflow enters the accommodating space from the dust suction port, and enters the second fan after passing through the filter.

Further, the maintenance station further includes a dust storage box for storing dust extracted from the second shell, and a dust extraction channel communicating with the dust storage box and the dust extraction port.

Further, a dust detection device is disposed in the dust extraction channel.

Further, an ultraviolet germicidal lamp is provided in the dust extraction channel and/or the dust storage box.

Further, the air discharge end of the first fan communicates with the air discharge port through an arc-shaped air channel or an inclined air channel.

Compared with the prior art, the beneficial effect of the present application is as follows: the intelligent cleaning system of the present application is provided with the dust extraction port and the air discharge port on the first shell of the maintenance station, and is provided with the dust outlet port and the air blowing port on the second shell of the sweeping robot. The air is discharged from the air discharge port by the first fan in the first shell, and enters into the second shell from the air blowing port, which applies a certain kinetic energy to the dust in the second shell to make the dust float up, and then the first fan is used to suck the dust from the dust outlet port into the maintenance station. As a result, the clearance rate of the dust box of the sweeping robot is improved, thereby ensuring that the interior of the dust box of the sweeping robot is completely clean and preventing mildew.

According to a second aspect, a purpose of the present application is to provide an intelligent cleaning system, which can effectively improve the clearance rate of a dust box of a sweeping robot, so as to ensure that an interior of the dust box of the sweeping robot is completely clean, prevent mildew, and avoid a large amount of bacteria in the cavity.

In order to achieve the above purpose, the present application provides the following technical solution: an intelligent cleaning system, including: a sweeping robot and a maintenance station configured to mate with the sweeping robot, the maintenance station including:

a first shell being provided with a dust extraction port;

a first fan arranged on the first shell, an air suction end of the first fan communicating with the dust extraction port so as to be used for extracting dust from the sweeping robot;

a cavity provided in the first shell, the cavity communicating with the dust extraction port through a dust extraction channel, a sterilization device being provided in the dust extraction channel and/or the cavity; and

a charging assembly disposed on the first shell and configured to charge the sweeping robot;

the sweeping robot including:

a second shell provided with a dust suction port and a dust outlet port, the dust outlet port being configured to mate with the dust extraction port; and

a working assembly arranged on the second shell to drive the sweeping robot to move and clean.

Further, the first shell is further provided with an air discharge port, and an air discharge end of the first fan communicates with the air discharge port; and wherein the second shell is provided with an air flowing port which is mated with the air discharge port.

Further, the second shell has a bottom surface; the dust outlet port and the air flowing port are arranged at opposite ends of the bottom surface, respectively.

Further, the bottom surface includes a first bottom surface and a second bottom surface; a height of the second bottom surface is higher than that of the first bottom surface in a vertical direction of the sweeping robot; the dust outlet port and the air flowing port are arranged on the first bottom surface, and the dust suction port is arranged on the second bottom surface.

Further, an air inlet valve is provided at the air flowing port; when the sweeping robot is in a working state, the air inlet valve closes the air flowing port; when the sweeping robot is mated with the maintenance station, the air inlet valve opens the air flowing port, so that the air flowing port communicates with the air discharge port.

Further, a dust discharge valve is provided at the dust outlet port; when the sweeping robot is in the working state, the dust discharge valve closes the dust outlet port; when the sweeping robot is mated with the maintenance station, the dust discharge valve opens the dust outlet port, so that the dust outlet port communicates with the dust extraction port.

Further, the second shell has an accommodating space; and when the sweeping robot is in a working state, the accommodating space is in a negative pressure state.

Further, the working assembly includes a ground brush assembly disposed at the dust suction port, a driving assembly driving the ground brush assembly to rotate, a moving assembly for moving the sweeping robot, and a second fan for making the accommodating space in a negative pressure state.

Further, the working assembly further includes a filter which is arranged between the second fan and the accommodating space; when the sweeping robot is in the working state, dust-carrying airflow enters the accommodating space from the dust suction port, and enters the second fan after passing through the filter.

Further, a dust detection device is provided in the dust extraction channel, the dust detection device includes a sensor and a microprocessor, the sensor is electrically connected to the microprocessor, and the microprocessor is electrically connected to the first fan.

Further, an ultraviolet germicidal lamp is disposed in the dust extraction channel and/or the cavity.

Further, the air discharge end of the first fan communicates with the air discharge port through an arc-shaped air channel or an inclined air channel.

Compared with the prior art, the beneficial effect of the present application is as follows: the intelligent cleaning system of the present application is provided with the dust extraction port and the air discharge port on the first shell of the maintenance station, and is provided with the dust outlet port and the air blowing port on the second shell of the sweeping robot. The air is discharged from the air discharge port by the first fan in the first shell, and enters into the second shell from the air blowing port, which applies a certain kinetic energy to the dust in the second shell to make the dust float up, and then the first fan is used to suck the dust from the dust outlet port into the maintenance station. As a result, the clearance rate of the dust box of the sweeping robot is improved, thereby ensuring that the interior of the dust box of the sweeping robot is completely clean and preventing mildew. At the same time, disposing the sterilization device in the cavity can prevent a large amount of bacteria from being generated in the cavity, thereby avoiding secondary pollution to the air.

According to a third aspect, a purpose of the present application is to provide a dust-full detection system and method for a dust-collecting bucket capable of automatically prompting a user to clean the dust-collecting bucket, which is convenient and quick to operate.

In order to achieve the above purpose, the present application provides the following technical solution: a dust-full detection system for a dust-collecting bucket, including:

an acquisition unit including a sensor and/or a detection circuit, the sensor and/or the detection circuit being configured to detect a parameter of the dust-collecting bucket;

a processing unit configured to receive the parameter and determine whether the parameter exceeds a preset threshold; and

an alarm unit configured to remind a user to perform a related operation;

wherein the processing unit instructs the alarm unit to send out indication information according to a determination result.

Further, when the dust-collecting bucket is working, the parameter is a parameter of a current and/or a parameter of a rotation speed of a dust extraction motor of the dust-collecting bucket.

Further, when the dust-collecting bucket is working, the parameter is a parameter of an air pressure in a dust bucket of the dust-collecting bucket.

Further, when the dust-collecting bucket is working, the parameter is a parameter of a weight of a dust bucket of the dust-collecting bucket.

Further, when the parameter exceeds the preset threshold, the alarm unit alarms to remind the user to clean the dust-collecting bucket.

Further, when the dust-collecting bucket is working, the parameter is a parameter of in-position time of a dust bucket of the dust-collecting bucket.

Further, when the dust-collecting bucket is working, the parameter is a parameter of working times of a dust extraction motor of the dust-collecting bucket.

Further, when the dust-collecting bucket is in operation, the parameter is a parameter of working times of a dust extraction motor within an in-position time of a dust bucket of the dust-collecting bucket.

According to a fourth aspect, the present application also provides a dust-full detection method for a dust-collecting bucket, including:

detecting a parameter of the dust-collecting bucket;
determining whether the parameter exceeds a preset threshold; and
sending indication information according to a determination result, and the indication information being alarm information.

Further, the method further includes: sending an indication message to remind a user to clean the dust-collecting bucket when the parameter exceeds the preset threshold.

The beneficial effects of the present application are: comparing the acquired parameter of the dust-collecting bucket with the preset threshold, if the parameter exceeds the preset threshold, a warning message will be issued to remind the user to clean the dust-collecting bucket, which is convenient and quick.

According to a fifth aspect, a purpose of the present application is to provide a cleaning control method for a dust box of a sweeping robot, a device and a storage medium, which can maintain the operation of its second fan during the dust extraction process, keep the dust box in a negative pressure state, so as to prevent the dust from being blown out from an dust inlet port of the sweeping robot during the dust extraction process.

To achieve the above purpose, the present application provides the following technical solution: a cleaning control method for a dust box of a sweeping robot, the sweeping robot configured to mate with a maintenance station, the maintenance station having a first fan, the sweeping robot having a second fan, the method including:

mating the sweeping robot with the maintenance station for charging;
starting the second fan first, and then starting the first fan to extract dust from the sweeping robot; or, starting the first fan and the second fan at the same time; and
when dust extraction is completed, turning off the first fan first, and then turning off the second fan; or, turning off the first fan and the second fan at the same time.

Further, the method further includes:
determining whether there is still dust drawn into the maintenance station; if not, turning off the first fan, and the dust extraction is completed; if yes, continuing the dust extraction.

Further, an air discharge end of the first fan communicates with the sweeping robot through an air channel, an air suction end of the first fan communicates with the sweeping robot through a dust extraction channel; a dust detection device is arranged in the dust extraction channel to detect whether there is still dust drawn into the dust extraction channel.

Further, the method further includes:
before performing the dust extraction, presetting a cleaning time threshold of the dust box; determining whether an actual working time of the first fan reaches the cleaning time threshold; and controlling the first fan to continue or stop working according to a determination result.

Further, the method further includes:

before presetting the cleaning time threshold of the dust box, obtaining a capacity of the dust box and a working flow of the first fan; and

calculating an ideal cleaning time of the dust box according to the capacity of the dust box and the working flow of the first fan; wherein the ideal cleaning time is:

$$T\_min = A/B;$$

where A is the capacity of the dust box, and B is the working flow of the first fan.

Further, the method further includes:

setting a clean-up working time of the first fan, the clean-up working time being longer than the ideal cleaning time; and
setting the clean-up working time as the cleaning time threshold of the dust box; wherein the clean-up working time is N times of the ideal cleaning time, and N is a positive integer.

Further, the first fan is connected with the dust box through an air channel, a flow sensor is disposed in the air channel, and the flow sensor is configured to detect the working flow of the first fan.

Further, the cleaning time threshold is a fixed dust extraction time.

The present application provides a cleaning control device for a dust box of a sweeping robot, the sweeping robot configured to mate with a maintenance station, the maintenance station having a first fan, the sweeping robot having a second fan, the device including:

a preset module configured to preset a cleaning time threshold of the dust box;
a determination module configured to determine whether there is still dust drawn into the maintenance station; or configured to determine whether an actual working time of the first fan has reached the cleaning time threshold after starting the first fan; and
a control module configured to control the first fan according to a determination result; or, configured to control the first fan and the second fan to continue or stop working.

The present application provides a cleaning control device for a dust box of a sweeping robot, the device including a processor and a memory, a program being stored in the memory, and the program being loaded and executed by the processor to implement the cleaning control method for the dust box of the sweeping robot as described above.

The present application provides a computer-readable storage medium, including a program stored in the storage medium; the program being executed by a processor to implement the cleaning control method for the dust box of the sweeping robot as described above.

Compared with the prior art, the beneficial effects of the present application are:

1) make the sweeping robot maintain the operation of its second fan during the dust extraction process, and keep the dust box in the negative pressure state, thereby preventing the dust from being blown out from the dust inlet port of the sweeping robot during the dust extraction process;
2) by installing the dust detection device in the dust extraction channel to detect whether the dust is inhaled, the first fan of the maintenance station can be prevented from idling, thereby avoiding energy waste and noise;
3) by setting the cleaning time threshold of the dust box, when the actual working time of the first fan reaches the cleaning time threshold, the first fan is turned off, which is convenient and intelligent;
4) by obtaining the capacity of the dust box and the working flow of the first fan, the ideal cleaning time of the first fan is calculated according to the ratio of the two. According to the ideal cleaning time, the clean-up working time of the first fan is set, which is fast and can avoid the idling of the first fan, thereby improving user experience.

According to a sixth aspect, a purpose of the present application is to provide a cleaning control method for a dust box of a sweeping robot, a device and a storage medium, which can intelligently determine whether the dust box is cleaned so as to improve user experience.

To achieve the above purpose, the present application provides the following technical solution:
a cleaning control method for a dust box of a sweeping robot, the sweeping robot configured to mate with a dust-collecting bucket, the sweeping robot having a dust box, the dust-collecting bucket having a dust extraction fan, the method including:

  presetting a cleaning time threshold required for cleaning the dust box;
  starting the dust extraction fan, and determining whether an actual working time of the dust extraction fan reaches the cleaning time threshold; and
  controlling the dust extraction fan to continue or stop working, according to the determination result.

Optionally, the method further includes:

  before presetting the cleaning time threshold required for cleaning the dust box, obtaining a capacity of the dust box, and obtaining an average working flow of the dust extraction fan according to the capacity of the dust box; and

  calculating an ideal cleaning time of the dust box according to the capacity of the dust box and the average working flow of the dust extraction fan; wherein the ideal cleaning time is:

$$T\_min = k*A/B;$$

  where k >=1 and is a natural number, A is the capacity of the dust box, and B is the average working flow of the dust extraction fan.

Optionally, the method further includes:

  setting a clean-up working time of the dust extraction fan, and the clean-up working time being longer than the ideal cleaning time; and

  setting the clean-up working time as a cleaning time threshold required for cleaning the dust box.

Optionally, the clean-up working time is N times the ideal cleaning time, where N is a positive integer.
Optionally, the dust extraction fan is connected with the dust box through an air channel, and a sensor is disposed in the air channel;
the sensor is configured to detect the average working flow of the dust extraction fan.
Optionally, the cleaning time threshold is a fixed dust extraction time.
The present application provides a cleaning control device for a dust box of a sweeping robot, the device including:

  a preset module configured to preset a cleaning time threshold of the dust box;
  a determination module configured to determine whether an actual working time of a dust extraction fan reaches the cleaning time threshold after the dust extraction fan is started; and
  a control module configured to control the dust extraction fan to continue or stop working according to a determination result.

The present application provides a cleaning control device for a dust box of a sweeping robot, the device including a processor and a memory, a program being stored in the memory, and the program being loaded and executed by the processor to implement the cleaning control method for the dust box of the sweeping robot as described above.
According to a fourth aspect, the present application provides a computer-readable storage medium, including a program stored in the storage medium, the program being executed by a processor to implement the cleaning control method for the dust box of the sweeping robot as described above.
The beneficial effects of the present application are: by setting the cleaning time threshold of the dust box, when the actual working time of the dust extraction fan reaches the cleaning time threshold, the dust extraction fan is turned off, which is convenient and intelligent; and
by obtaining the capacity of the dust box and the working flow of the dust extraction fan, the ideal cleaning time of the dust extraction fan is calculated according to the ratio of the two. According to the ideal cleaning time, the clean-up working time of the dust extraction fan is set, which is fast and can avoid the idling of the dust extraction fan, thereby improving the user experience.
The above description is only an overview of the technical solutions of the present application. In order to have a clearer understanding of the technical means of the present application and to implement them in accordance with the contents of the specification, the following detailed description is given with reference to the preferred embodiments of the present application and the accompanying drawings.

## BRIEF DESCRIPTION OF DRAWINGS

FIG. 1 is a schematic structural view of an intelligent cleaning system according to an embodiment of the present application;

FIG. 2 is a schematic structural view of a sweeping robot according to an embodiment of the present application;

FIG. 3 is a structural cross-sectional view of a sweeping robot according to an embodiment of the present application;

FIG. 4 is a schematic structural view of an intelligent cleaning system according to an embodiment of the present application;

FIG. 5 is a schematic structural view of a sweeping robot according to an embodiment of the present application;

FIG. 6 is a structural cross-sectional view of a sweeping robot according to an embodiment of the present application;

FIG. 7 is a structural block diagram of a dust-full detection system of a dust-collecting bucket of the present application;

FIG. 8 is a flowchart of a dust-full detection method of a dust-collecting bucket of the present application;

FIG. 9 is a circuit schematic diagram of an acquisition unit of the present application;

FIG. 10 is a cleaning control method for a dust box of a sweeping robot provided by an embodiment of the present application;

FIG. 11 is a cleaning control method for a dust box of a sweeping robot provided by an embodiment of the present application;

FIG. 12 is a cleaning control method for a dust box of a sweeping robot provided by an embodiment of the present application;

FIG. 13 is a cleaning control device for a dust box of a sweeping robot provided by an embodiment of the present application;

FIG. 14 is a cleaning control device for a dust box of a sweeping robot provided by an embodiment of the present application;

FIG. 15 is a kind of intelligent cleaning system provided by an embodiment of the present application;

FIG. 16 is a kind of intelligent cleaning system provided by an embodiment of the present application;

FIG. 17 is a kind of intelligent cleaning system provided by an embodiment of the present application;

FIG. 18 is a cleaning control method for a dust box of a sweeping robot provided by an embodiment of the present application;

FIG. 19 is a cleaning control device for a dust box of a sweeping robot provided by an embodiment of the present application; and

FIG. 20 is a cleaning control device for a dust box of a sweeping robot provided by another embodiment of the present application.

## DETAILED DESCRIPTION

Specific implementations of the present application will be described in further detail below with reference to the accompanying drawings and embodiments. The following examples are used to illustrate the present application, but are not intended to limit the scope of the present application.

It should be noted that terms such as "top", "bottom", "left", "right", "inner" and "outer" in the present application are only used to describe the present application with reference to the drawings, and are not used as limiting terms.

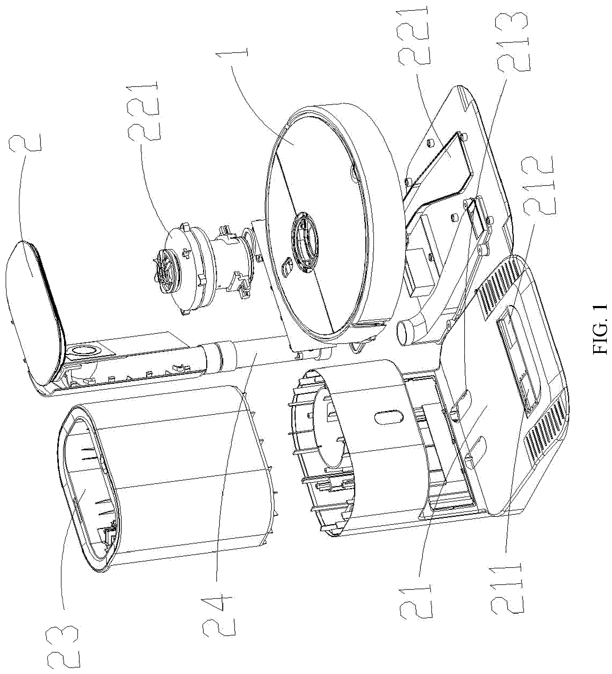

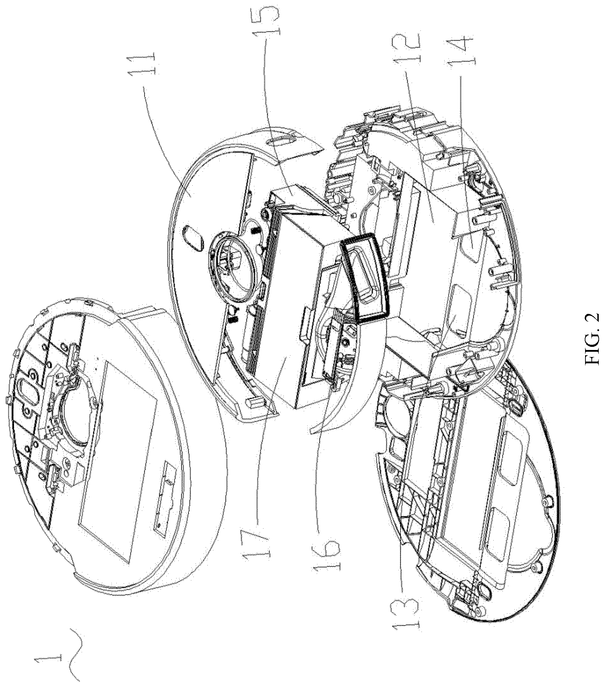

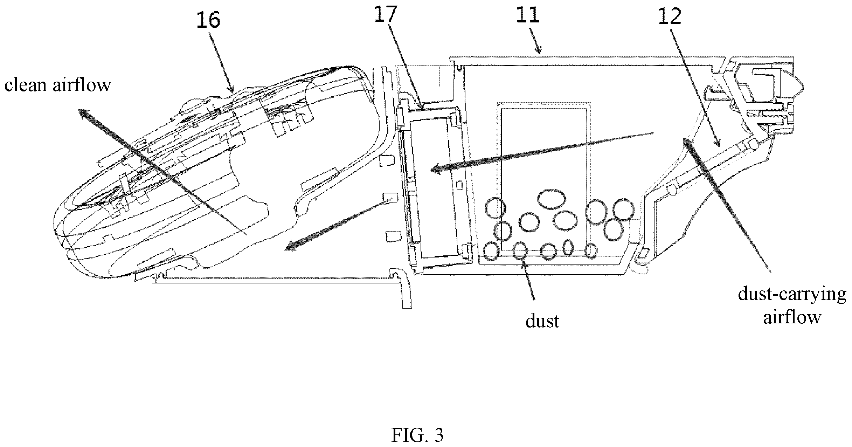

Referring to FIG. 1 to FIG. 3, an intelligent cleaning system shown in a preferred embodiment of the present application includes a sweeping robot 1 and a maintenance station 2 configured to mate with the sweeping robot 1. The maintenance station 2 in this embodiment can be used to perform functions such as charging and dust extraction for the sweeping robot 1.

The maintenance station 2 includes a first shell 21, a first fan 22 disposed in the first shell 21, and a charging assembly. The first shell 21 is provided with a dust extraction port 211 and an air discharge port 212. An air discharge end and an air suction end of the first fan 22 communicate with the air discharge port 212 and the dust extraction port 211, respectively. In this embodiment, in order to reduce the loss of air volume, the air discharge end of the first fan 22 and the air discharge port 212 are communicated with each other through an inclined channel 221. Similarly, the air suction end and the dust extraction port 211 can also be communicated with each other through an inclined channel. Indeed, in other embodiments, an arc-shaped air channel may be an arc-shaped channel or the like.

In this embodiment, the charging assembly and the sweeping robot 1 generally use a charging elastic sheet 213 for contact charging. The charging elastic sheet 213 and the first fan 22 are both in the prior art, which will not be described in detail here.

The sweeping robot 1 includes a second shell 11 and a working assembly disposed in the second shell 11. The second shell 11 is provided with a dust suction port 12, a dust outlet port 13 and an air blowing port 14. The dust suction port 12 is a conventional structure of the sweeping robot 1, and is usually provided at the bottom of the second shell 11. In this embodiment, the dust outlet port 13 and the air blowing port 14 are disposed at opposite ends of the second shell 11. The dust outlet port 13 is configured to mate with the dust extraction port 211. The air blowing port 14 is configured to mate with the air discharge port 212. The first fan 22 discharges the air from the air discharge port 212 and enters the second shell 11 from the air blowing port 14, which applies a certain kinetic energy to the dust in the second shell 11 to make the dust float up, and then the first fan 22 is used to suck the dust from the dust outlet port into the maintenance station 2. As a result, the clearance rate of the dust box of the sweeping robot 1 is improved. In addition, the working assembly is also provided in the second shell 11, so that the sweeping robot 1 can move and clean.

Specifically, the second shell 11 has a bottom surface and an accommodating space 110. The dust outlet port 13 and the air blowing port 14 are arranged at opposite ends of the bottom surface, respectively. More preferably, the bottom surface includes a first bottom surface 111 and a second bottom surface 112. In a vertical direction of the sweeping robot 1, a height of the second bottom surface 112 is higher than that of the first bottom surface 111, and the dust outlet port 13 and the air blowing port 14 are arranged on the first bottom surface 111. The dust suction port 12 is arranged on the second bottom surface 112. This arrangement ensures that the dust sucked into the second shell 11 will not easily fall out from the dust suction port 12. Meanwhile, the sweeping robot 1 of this embodiment is provided with an air inlet valve at the air blowing port 14, and a dust discharge valve is provided at the dust outlet port 13. When the sweeping robot 1 is in a working state, the accommodating space 110 is in a negative pressure state, the air inlet valve closes the air blowing port 14, the dust discharge valve closes the dust outlet port 13, and dust is sucked from the dust suction port 12. When the sweeping robot 1 is mated with the maintenance station 2, the air inlet valve opens the air blowing port 14, and the dust discharge valve opens the dust outlet port 13. As a result, the air blowing port 14 and the dust outlet port 13 communicate with the air discharge port 212 and the dust extraction port 211, respectively, so that the dust extraction work is performed.

In this embodiment, the working assembly includes a ground brush assembly 15 disposed at the dust suction port 12, a driving assembly for driving the ground brush assembly 15 to rotate, a moving assembly for moving the sweeping robot 1, and a second fan 16 for keeping the accommodating space 110 in a negative pressure state. The working assembly further includes a filter 17 which is disposed between the second fan 16 and the accommodating space 110. When the sweeping robot 1 is in the working state, the second fan 16 starts to work, so that the accommodating space 110 is in the negative pressure state. Under the action of the ground brush assembly 15 and the second fan 16, the external dust-carrying airflow enters the accommodating space 110 from the dust suction port 12. After passing through the filter 17, the dust stays in the accommodating space 110, and the airflow enters the second fan 16.

Of course, in order to realize the sealing of the accommodating space 110, the second shell 11 is also provided with an end cover, and a sealing structure, etc., which are in the prior art and will not be described here.

In the intelligent cleaning system of the present application, the maintenance station 2 is further provided with a dust storage box 23 for storing the dust extracted from the second shell 11, and a dust extraction channel 24 communicating with the dust storage box 23 and the dust extraction port 211. A dust detection device is disposed in the dust extraction channel 24. When the charging elastic sheet on the sweeping robot is in contact with the charging elastic sheet 213 of the maintenance station 2, the first fan 22 of the maintenance station 2 starts to work so as to perform a dust extraction action. The dust in the dust box of the sweeping robot enters the dust storage box 23 through the dust extraction channel 24. When the dust detection device detects that no dust enters the dust extraction passage 24, the first fan 22 of the maintenance station 2 stops working. In this embodiment, the dust detection device includes a sensor and a microprocessor. The sensor is electrically connected to the microprocessor. The microprocessor is electrically connected to the first fan 22. The sensor is used to determine whether there is still dust drawn into the dust extraction channel 24, and then the microprocessor receives a sensor signal and controls the on or off of the first fan 22. When the sensor determines that no dust is drawn into the dust extraction channel 24, the first fan 22 is turned off to avoid idling, avoid wasting energy and avoid generating noise.

In this embodiment, an ultraviolet germicidal lamp is also provided in the dust storage box 23. Certainly, in other embodiments, the ultraviolet germicidal lamp may also be disposed in the dust extraction channel 24. However, disposing the ultraviolet germicidal lamp in the dust storage box 23 can ensure complete sterilization. When the charging elastic sheet of the sweeping robot is in contact with the charging elastic sheet 213 of the maintenance station 2, the first fan 22 of the maintenance station 2 starts to work so as to perform the dust extraction action. At this time, the dust storage box 23 and/or the ultraviolet germicidal lamp in the dust extraction channel 24 lights up. The dust enters the dust box of the maintenance station 2 through the dust extraction channel 24. This design can prevent a large amount of bacteria from being generated in the dust storage box 23 of the maintenance station 2, thereby avoiding secondary pollution to the air.

In summary, the intelligent cleaning system of the present application is provided with the dust extraction port and the air discharge port on the first shell of the maintenance station, and is provided with the dust outlet port and the air blowing port on the second shell of the sweeping robot. The air is discharged from the air discharge port by the first fan in the first shell, and enters into the second shell from the air blowing port, which applies a certain kinetic energy to the dust in the second shell to make the dust float up, and then the first fan is used to suck the dust from the dust outlet port into the maintenance station. As a result, the clearance rate of the dust box of the sweeping robot is improved, thereby ensuring that the interior of the dust box of the sweeping robot is completely clean and preventing mildew.

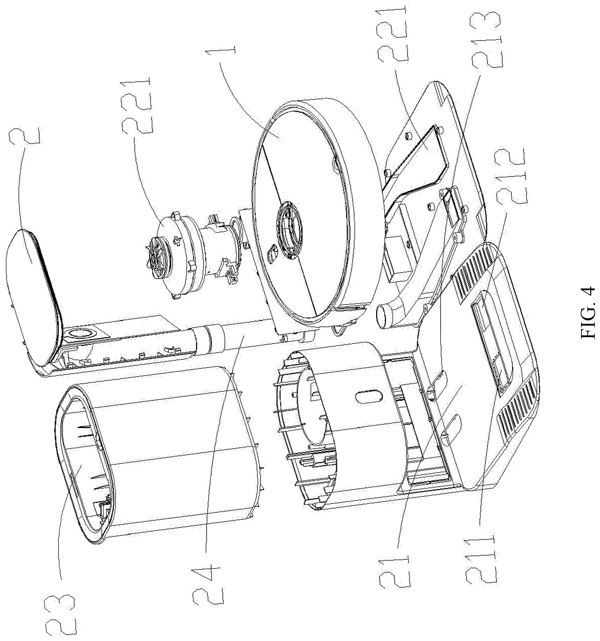

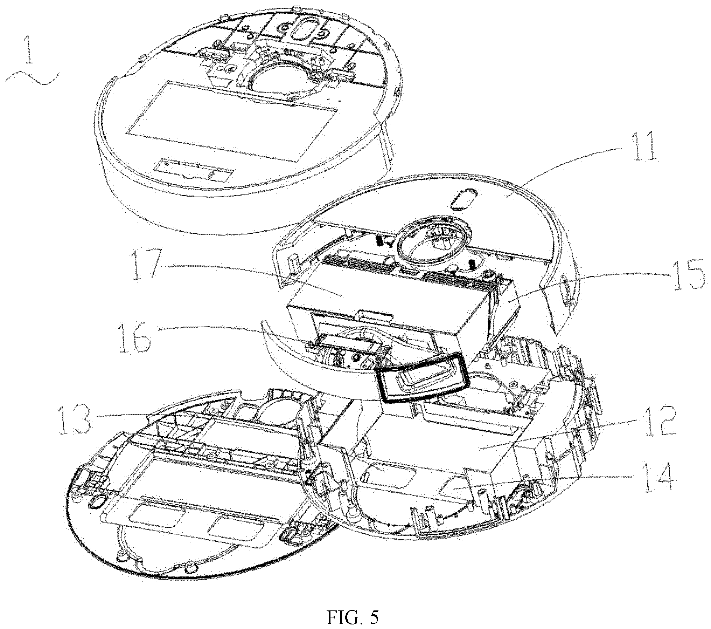

Referring to FIG. 4 to FIG. 6, an intelligent cleaning system shown in a preferred embodiment of the present application includes a sweeping robot 1 and a maintenance station 2 configured to mate with the sweeping robot 1. The maintenance station 2 in this embodiment can be used to perform functions such as charging and dust extraction for the sweeping robot 1.

The maintenance station 2 includes a first shell 21, a first fan 22 disposed in the first shell 21, and a charging assembly. The first shell 21 is provided with a dust extraction port 211 and an air discharge port 212. An air discharge end and an air suction end of the first fan 22 communicate with the air discharge port 212 and the dust extraction port 211, respectively. In this embodiment, in order to reduce the loss of air volume, the air discharge end of the first fan 22 and the air discharge port 212 are communicated with each other through an inclined channel 221. Similarly, the air suction end and the dust extraction port 211 can also be communicated with each other through an inclined channel. Indeed, in other embodiments, an arc-shaped air channel may be an arc-shaped channel or the like.

In this embodiment, the charging assembly and the sweeping robot 1 generally use a charging elastic sheet 213 for contact charging. The charging elastic sheet 213 and the first fan 22 are both in the prior art, which will not be described in detail here.

The sweeping robot 1 includes a second shell 11 and a working assembly disposed in the second shell 11. The second shell 11 is provided with a dust suction port 12, a dust outlet port 13 and an air blowing port 14. The dust suction port 12 is a conventional structure of the sweeping robot 1, and is usually provided at the bottom of the second shell 11. In this embodiment, the dust outlet port 13 and the air blowing port 14 are disposed at opposite ends of the second shell 11. The dust outlet port 13 is configured to mate with the dust extraction port 211. The air blowing port 14 is configured to mate with the air discharge port 212. The first fan 22 discharges the air from the air discharge port 212 and enters the second shell 11 from the air blowing port 14, which applies a certain kinetic energy to the dust in the second shell 11 to make the dust float up, and then the first fan 22 is used to suck the dust from the dust outlet port into the maintenance station 2. As a result, the clearance rate of the dust box of the sweeping robot 1 is improved. In addition, the working assembly is also provided in the second shell 11, so that the sweeping robot 1 can move and clean.

Specifically, the second shell 11 has a bottom surface and an accommodating space 110. The dust outlet port 13 and the air blowing port 14 are arranged at opposite ends of the bottom surface, respectively. More preferably, the bottom surface includes a first bottom surface 111 and a second bottom surface 112. In a vertical direction of the sweeping robot 1, a height of the second bottom surface 112 is higher than that of the first bottom surface 111, and the dust outlet port 13 and the air blowing port 14 are arranged on the first bottom surface 111. The dust suction port 12 is arranged on the second bottom surface 112. This arrangement ensures that the dust sucked into the second shell 11 will not easily fall out from the dust suction port 12. Meanwhile, the sweeping robot 1 of this embodiment is provided with an air inlet valve at the air blowing port 14, and a dust discharge valve is provided at the dust outlet port 13. When the sweeping robot 1 is in a working state, the accommodating space 110 is in a negative pressure state, the air inlet valve closes the air blowing port 14, the dust discharge valve closes the dust outlet port 13, and dust is sucked from the dust suction port 12. When the sweeping robot 1 is mated with the maintenance station 2, the air inlet valve opens the air blowing port 14, and the dust discharge valve opens the dust outlet port 13. As a result, the air blowing port 14 and the dust outlet port 13 communicate with the air discharge port 212 and the dust extraction port 211, respectively, so that the dust extraction work is performed.

In this embodiment, the working assembly includes a ground brush assembly 15 disposed at the dust suction port 12, a driving assembly for driving the ground brush assembly 15 to rotate, a moving assembly for moving the sweeping robot 1, and a second fan 16 for keeping the accommodating space 110 in a negative pressure state. The working assembly further includes a filter 17 which is disposed between the second fan 16 and the accommodating space 110. When the sweeping robot 1 is in the working state, the second fan 16 starts to work, so that the accommodating space 110 is in the negative pressure state. Under the action of the ground brush assembly 15 and the second fan 16, the external dust-carrying airflow enters the accommodating space 110 from the dust suction port 12. After passing through the filter 17, the dust stays in the accommodating space 110, and the airflow enters the second fan 16.

Of course, in order to realize the sealing of the accommodating space 110, the second shell 11 is also provided with an end cover, and a sealing structure, etc., which are in the prior art and will not be described here.

In the intelligent cleaning system of the present application, the maintenance station 2 is further provided with a cavity 23 for storing the dust extracted from the second shell 11, and a dust extraction channel 24 communicating with the cavity 23 and the dust extraction port 211. Preferably, one or more dust bag structures for collecting dust, or one or more dust box structures for easy removal can be arranged in the cavity 23. The specific arrangement manner of the one or more dust bag structures or the one or more dust box structures can be selected according to actual needs. A dust detection device is disposed in the dust extraction channel 24. When the charging elastic sheet on the sweeping robot is in contact with the charging elastic sheet 213 of the maintenance station 2, the first fan 22 of the maintenance station 2 starts to work so as to perform a dust extraction action. The dust in the dust box of the sweeping robot enters the cavity 23 through the dust extraction channel 24. When the dust detection device detects that no dust enters the dust extraction passage 24, the first fan 22 of the maintenance station 2 stops working. In this embodiment, the dust detection device includes a sensor and a microprocessor. The sensor is electrically connected to the microprocessor. The microprocessor is electrically connected to the first fan 22. The sensor is used to determine whether there is still dust drawn into the dust extraction channel 24, and then the microprocessor receives a sensor signal and controls the on or off of the first fan 22. When the sensor determines that no dust is drawn into the dust extraction channel 24, the first fan 22 is turned off to avoid idling, avoid wasting energy and avoid generating noise.

In this embodiment, a sterilization device is also provided in the cavity 23. Preferably, the sterilization device is an ultraviolet germicidal lamp. Certainly, in other embodiments, the ultraviolet germicidal lamp may also be disposed in the dust extraction channel 24. However, disposing the ultraviolet germicidal lamp in the cavity 23 can ensure complete sterilization. When the charging elastic sheet of the sweeping robot is in contact with the charging elastic sheet 213 of the maintenance station 2, the first fan 22 of the maintenance station 2 starts to work so as to perform the dust extraction action. At this time, the cavity 23 and/or the ultraviolet germicidal lamp in the dust extraction channel 24 lights up. The dust enters the dust box of the maintenance station 2 through the dust extraction channel 24. This design can prevent a large amount of bacteria from being generated in the cavity 23 of the maintenance station 2, thereby avoiding secondary pollution to the air.

In summary, the intelligent cleaning system of the present application is provided with the dust extraction port and the air discharge port on the first shell of the maintenance station, and is provided with the dust outlet port and the air blowing port on the second shell of the sweeping robot. The air is discharged from the air discharge port by the first fan in the first shell, and enters into the second shell from the air blowing port, which applies a certain kinetic energy to the dust in the second shell to make the dust float up, and then the first fan is used to suck the dust from the dust outlet port into the maintenance station. As a result, the clearance rate of the dust box of the sweeping robot is improved, thereby ensuring that the interior of the dust box of the sweeping robot is completely clean and preventing mildew. At the same time, disposing the sterilization device in the cavity can prevent a large amount of bacteria from being generated in the cavity, thereby avoiding secondary pollution to the air.

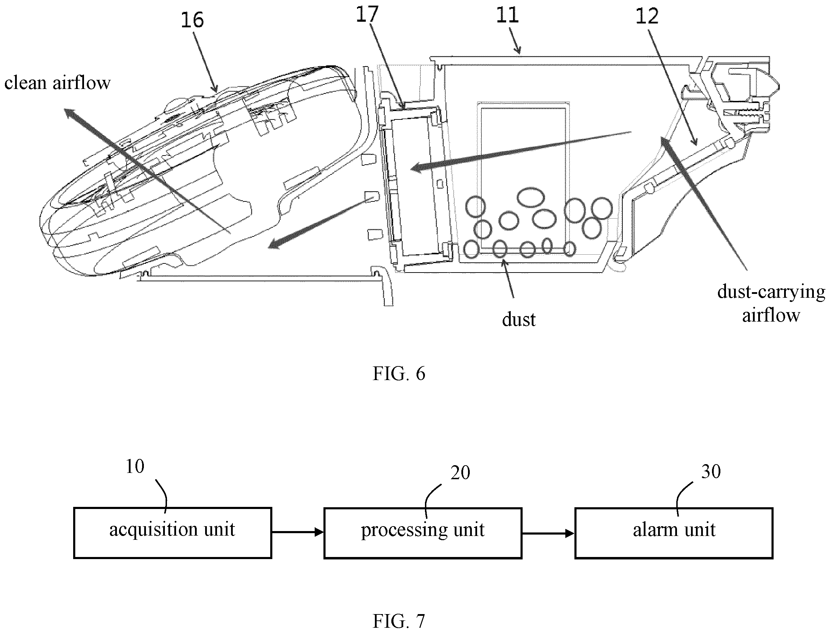

Referring to FIG. 7, a dust-full detection system for a dust-collecting bucket in a preferred embodiment of the present application is configured to detect whether the dust bucket in the dust-collecting bucket needs to be cleaned. If cleaning is required, a warning will be issued to remind the user, which is convenient and quick. Among them, the dust-collecting bucket is mated with the sweeping robot. The dust-collecting bucket may be integrated with the sweeping robot; or, the dust-collecting bucket and the sweeping robot may be arranged separately, which is not specifically limited here, and depends on the actual situation. When the dust-collecting bucket is integrated with the sweeping robot, since the capacity of the dust-collecting bucket is limited, it needs to be actively cleaned regularly. When the dust-collecting bucket and the sweeping robot are arranged separately, the sweeping robot needs to move to a place where the sweeping robot is mated with the dust-collecting bucket so as to perform work. In this embodiment, the dust-collecting bucket and the sweeping robot are arranged separately. The sweeping robot is a conventional structure, which will not be described in detail in the present application.

The dust-collecting bucket includes a shell, a dust bucket disposed in the shell, and a dust suction mechanism for connecting the dust bucket and mating with the sweeping robot. The dust suction mechanism includes a dust extraction motor, a pipe for connecting the dust bucket and the sweeping robot, a dust-collecting box or a dust-collecting bag for storing particulate matter, and a filtering mechanism for filtering the airflow. When the sweeping robot is mated with the dust-collecting bucket, the dust extraction motor inside the dust-collecting bucket rotates, and the dust in the dust box of the sweeping robot is sucked into the dust-collecting bucket, which is convenient and quick. Among them, the filtering mechanism is a conventional structure, which may include a filter screen, a cyclone separator, an air outlet HYPA, etc., which will not be described in detail here.

The dust-collecting bucket also includes an acquisition unit 10, a processing unit 20 and an alarm unit 30 which are arranged in or on the shell. Specifically, the acquisition unit 10 is used to detect a parameter of the dust-collecting bucket. The processing unit 20 is used for determining whether the parameter exceeds a preset threshold. The alarm unit 30 is used to remind the user to perform related operations. The processing unit 20 instructs the alarm unit 30 to send out indication information according to the determination result. Specifically, when the parameter exceeds the preset threshold, the alarm unit 30 alarms to remind the user to clean the dust-collecting bucket. In this embodiment, the acquisition unit 10 is a detection circuit and/or a sensor, which is used to sense the state inside the dust-collecting bucket and convert the detection result into an electrical signal. The processing unit 20 is a microprocessor, which receives and analyzes the electrical signal sent by the acquisition unit 10 to control the dust-collecting bucket to perform different actions. The alarm unit 30 is one or more of a light signal alarm, a sound signal alarm and a screen display. Alternatively, the alarm unit 30 is a terminal device accessing the network. Certainly, in other embodiments, the acquisition unit 10 can also be other, and the processing unit 20 and the alarming unit 30 can also be other ones, which are not specifically limited here, and are determined according to the actual situation.

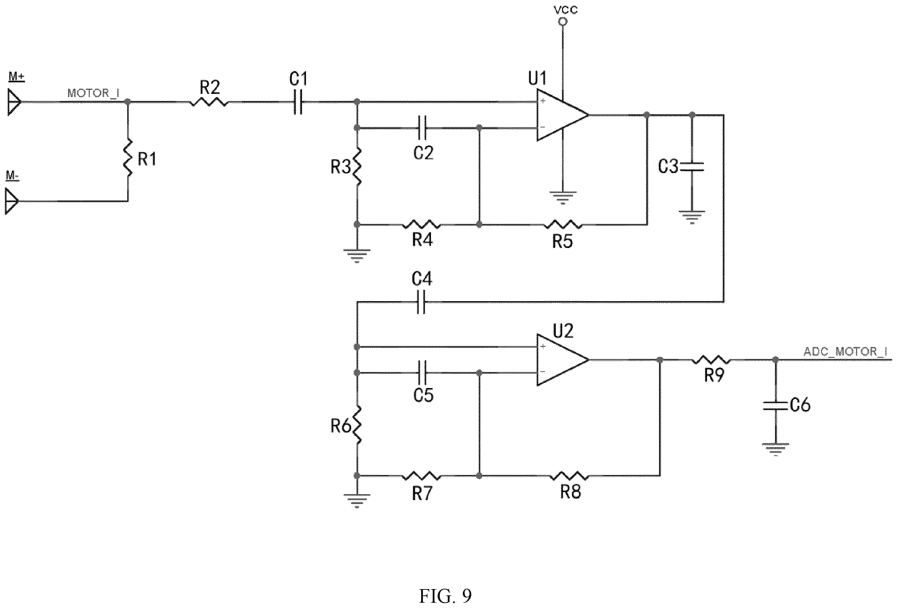

Referring to FIG. 9, it shows a schematic circuit diagram of the acquisition unit 10 as a detection circuit. Wherein, the detection circuit includes a sampling resistor for sampling the parameter of the dust-collecting bucket, and a primary amplification module and a secondary amplification module for amplifying the parameter. The parameter is sent to the processing unit 20 after being amplified in two stages. Two poles of the dust extraction fan are connected through a first resistor R1, and are connected to one end of the sampling resistor R2. The other end of the sampling resistor R2 is connected to one end of a first capacitor C1. The other end of the first capacitor C1 is connected to the primary amplification module. The primary amplification module and the secondary amplification module are connected through a fourth capacitor C4.

The primary amplification module includes a first amplifier U1. The other end of the first capacitor C1 is connected to a forward input end of the first amplifier U1. A reverse input end of the first amplifier U1 is connected to one end of a second capacitor C2. The other end of the second capacitor C2 is connected to one end of a third resistor R3 and then connected to a connection end of the first capacitor C1 and the first amplifier U1. The other end of the third resistor R3 is connected to one end of a fourth resistor R4 and then grounded. The other end of the fourth resistor R4 is connected to the second capacitor C2 and one end of a fifth resistor R5. The other end of the fifth resistor R5 is connected to one end of a third capacitor C3 and an output end of the first amplifier U1. The other end of the third capacitor C3 is grounded.

The secondary amplification module includes a second amplifier U2. One end of the fourth capacitor C4 is connected to the output end of the first amplifier U1, and the other end of the fourth capacitor C4 is connected to a forward input end of the second amplifier U2. A reverse input end of the second amplifier U2 is connected to one end of a fifth capacitor C5. The other end of the fifth capacitor C5 is connected to one end of a sixth resistor R6. The other end of the sixth resistor R6 is connected to one end of a seventh resistor R7 and then grounded. The other end of the seventh resistor R7 is connected to the fifth capacitor C5 and one end of an eighth resistor R8. The other end of the eighth resistor R8 is connected to one end of a ninth resistor R9. The other end of the ninth resistor R9 is connected to one end of a sixth capacitor C6. The other end of the sixth capacitor C6 is grounded.

When the dust-collecting bucket is working, the parameter is a parameter of a current of a dust extraction motor of the dust-collecting bucket. Correspondingly, in this embodiment, the acquisition unit 10 is a detection circuit. The detection circuit is used for acquiring the parameter of the current of the dust-collecting bucket and then sending the parameter of the current to a microprocessor. Specifically, when the dust-collecting bucket is empty, the dust extraction motor in the dust-collecting bucket absorbs dust from the dust box of the sweeping robot. At this time, the load of the dust extraction motor is relatively large, and accordingly, the current of the dust extraction motor is the largest. As the dust and debris gradually increase, they adhere to an air outlet HYPA of the dust-collecting bucket, which will cause the air outlet HYPA to be blocked. As a result, the load of the dust extraction motor becomes smaller, and the current of the dust extraction motor becomes smaller. When the debris and the dust completely block the air outlet HYPA, the dust extraction motor is equivalent to no load, and the current of the dust extraction motor is the smallest. Therefore, the preset threshold in the microprocessor is a value close to the minimum value of the current of the extraction motor. When the detection circuit detects that the current of the dust extraction motor is less than the preset threshold, the alarm unit 30 is activated to remind the user to clean the dust-collecting bucket.

Alternatively, when the dust-collecting bucket is working, the parameter is a parameter of a rotation speed of the dust extraction motor of the dust-collecting bucket. Correspondingly, in this embodiment, the acquisition unit 10 is a Hall sensor, a magnet, and a detection circuit electrically connected to the Hall sensor. Wherein, the magnet can be arranged on the dust extraction motor. The Hall sensor is arranged on one side of the magnet to detect the rotation speed of the dust extraction motor. The Hall sensor and the magnet are mutually induced to detect the parameter of the rotation speed of the dust extraction motor. The detection circuit acquires the parameter of the rotation speed and sends the parameter of the rotation speed to a microprocessor. Specifically, when the dust-collecting bucket is empty, the dust extraction motor in the dust-collecting bucket absorbs dust from the dust box of the sweeping robot, and the load of the dust extraction motor is relatively large at this time. Correspondingly, the rotation speed of the dust extraction motor is slow so as to maintain a constant power to work. As the dust and debris build up, the weight inside the dust bucket will increase. The particles in the dust bucket block the air outlet, and the dust extraction motor has a small load. In order to keep the dust extraction motor working at a constant power, the rotation speed of the dust extraction motor is increased. Therefore, the preset threshold in the microprocessor is a value close to the maximum rotation speed of the dust extraction motor. When the detection circuit detects that the rotation speed of the dust extraction motor is greater than the preset threshold, the alarm unit 30 is activated to remind the user to clean the dust-collecting bucket.

Alternatively, when the dust-collecting bucket is working, the parameter is a parameter of an air pressure in the dust bucket of the dust-collecting bucket. Correspondingly, in this embodiment, the acquisition unit 10 is an air pressure sensor disposed in the shell and a detection circuit electrically connected to the air pressure sensor. The air pressure sensor is used to detect the parameter of the air pressure in the shell. The detection circuit acquires the parameter of the air pressure and sends the parameter of the air pressure to a microprocessor. Specifically, when the dust-collecting bucket is empty, at this time, the dust-collecting bucket does not start the dust extraction motor. An interior of the dust-collecting bucket communicates to the outside atmosphere. The air pressure sensor collects the reference air pressure of the atmosphere. At this time, the collected air pressure value is the largest, and the air pressure value is an initial reference air pressure. As the dust and debris gradually increase, there will be more and more dust and fine debris in the dust-collecting bucket, and the air pressure value of the current air pressure collected by the air pressure sensor will become smaller and smaller. Therefore, the microprocessor makes a difference according to the initial reference air pressure collected by the air pressure sensor and the currently collected air pressure. A preset threshold is stored in the microprocessor. When the difference is greater than the preset threshold, it is considered that the dust-collecting bucket is seriously blocked, that is, the dust box is full of dust, so that the alarm unit 30 is activated to remind the user to clean the dust-collecting bucket.

Alternatively, when the dust-collecting bucket is working, the parameter is a parameter of a weight of the dust bucket of the dust-collecting bucket. Correspondingly, in this embodiment, the acquisition unit 10 is a weight sensor and a detection circuit electrically connected to the weight sensor. Wherein, the weight sensor is arranged on the dust bucket. The weight sensor detects the weight of the dust bucket. The detection circuit obtains the parameter of the weight and sends the parameter of the rotation speed to the microprocessor. Specifically, when the dust-collecting bucket is empty and the dust extraction motor in the dust-collecting bucket absorbs the dust of the dust box of the sweeping robot, the weight of the dust-collecting bucket is the lightest at this time. As the dust and debris gradually increase, the weight in the dust bucket will increase, causing the dust bucket to become heavier and heavier. Therefore, the preset threshold within the microprocessor is a value close to the maximum weight of the dust bucket. When the parameter of the weight detected by the detection circuit is greater than the preset threshold, the alarm unit 30 is activated to remind the user to clean the dust-collecting bucket.

Alternatively, when the dust-collecting bucket is working, the parameter is a parameter of in-position time of the dust bucket of the dust-collecting bucket. Correspondingly, in this embodiment, the acquisition unit 10 is an in-position sensor and a detection circuit electrically connected to the in-position sensor. Wherein, the in-position sensor is arranged on the shell. The in-position sensor detects whether the dust bucket is in the shell, and the detection circuit obtains the time parameter of the dust bucket in the shell and sends the time parameter to a microprocessor. Specifically, when the user cleans the dust bucket, the in-position sensor cannot detect the dust bucket and cannot send a signal to the microprocessor. After the user finishes cleaning the dust bucket, the in-position sensor detects the dust bucket again and continues to send signals to the microprocessor. The microprocessor records the time interval of the received signals from the in-position sensor, and then acquires, analyzes and learns the time and frequency of the user cleaning the dust bucket. The microprocessor can even acquire the user's cleaning habits (determined by the timing of the received signals) to set a time threshold. The time threshold can be any time, and is determined according to the cleaning habits acquired by the microprocessor. When a certain time is close to the time threshold, the microprocessor controls the alarm unit to issue an indication to remind the user to clean the dust bucket.

Alternatively, when the dust-collecting bucket is working, the parameter is a parameter of the working times of the dust extraction motor of the dust-collecting bucket. In the microprocessor, the maximum volume of the dust bucket of the dust-collecting bucket is set as A, and the maximum volume of the dust box of the sweeping robot is set as B. Therefore, a volume ratio of the dust bucket and the dust box is N=A/B. When the sweeping robot detects that the dust box is full, it returns to the dust-collecting bucket so as to mate with the dust-collecting bucket. The dust extraction motor starts to collect the dust debris in the dust box into the dust bucket, and makes the microprocessor store the work log. When the working frequency of the dust extraction motor is close to or equal to N, the microprocessor controls the alarm unit to issue an instruction to remind the user to clean the dust bucket.

Alternatively, when the dust-collecting bucket is working, the parameter is a parameter of the working times of the dust extraction motor within the in-position time of the dust bucket of the dust-collecting bucket. Correspondingly, in this embodiment, the acquisition unit 10 is an in-position sensor and a detection circuit electrically connected to the in-position sensor. Wherein, the in-position sensor is arranged on the shell. The in-position sensor detects whether the dust bucket is in the shell, and the detection circuit obtains the time parameter of the dust bucket in the shell and sends the time parameter to a microprocessor. In the microprocessor, the maximum volume of the dust bucket of the dust-collecting bucket is set as A, and the maximum volume of the dust box of the sweeping robot is set as B. Therefore, a volume ratio of the dust bucket and the dust box is N=A/B. Different from the above embodiment, the reminder in this embodiment is more precise. During the in-position time of the dust bucket, the maximum number of the working times of the dust extraction motor is N. When the sweeping robot detects that the dust box is full, it returns to the dust bucket so as to mate with the dust bucket. The dust extraction motor starts to collect the dust debris in the dust box into the dust bucket, and makes the microprocessor store the work log. When the working frequency of the dust extraction motor is close to or equal to N, the microprocessor controls the alarm unit to issue an instruction to remind the user to clean the dust bucket.

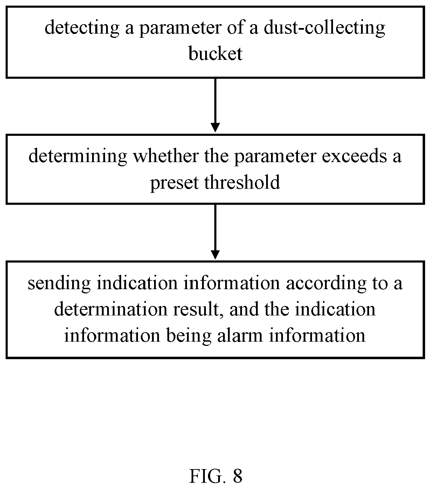

Referring to FIG. 8, the present application also provides a dust-full detection method for a dust-collecting bucket, including:

  detecting a parameter of the dust-collecting bucket;

  determine whether the parameter exceeds a preset threshold; and

  sending indication information according to a determination result, and the indication information being alarm information. Specifically, when the parameter exceeds the preset threshold, an indication message is sent to remind the user to clean the dust-collecting bucket.

In summary, comparing the acquired parameter of the dust-collecting bucket with the preset threshold, if the parameter exceeds the preset threshold, a warning message will be issued to remind the user to clean the dust-collecting bucket, which is convenient and quick.

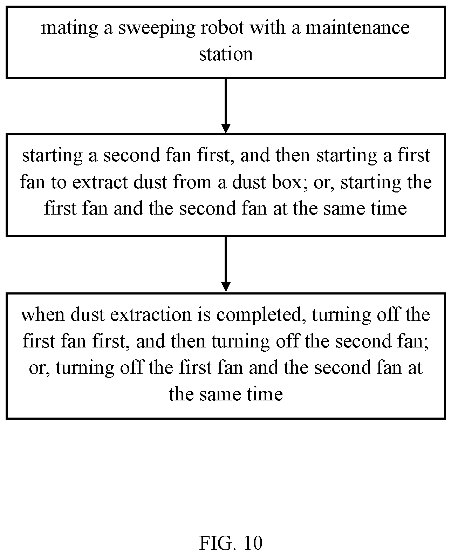

FIG. 10 is a cleaning control method for a dust box of a sweeping robot provided by an embodiment of the present application, which is applicable to a sweeping machine system. The sweeping robot is mated with the maintenance station to extract the dust from its dust box. The sweeping robot and the maintenance station can be arranged in an integrated manner or separately, depending on the actual situation, which is not specifically limited here. When the dust box of the sweeping robot is full of dust and debris, the sweeping robot docks with the maintenance station to clean the dust box.

The maintenance station includes a shell, a dust bucket disposed in the shell, and a first fan disposed in the shell. Wherein, the air discharge end and the air suction end of the first fan are communicated with the dust box through the air channel and the dust extraction channel, respectively. The working principle of the first fan is as follows: the air discharge end of the first fan blows the air from the air channel into the dust box, and applies a certain kinetic energy to the dust in the dust box. After the dust is floated up, the first fan draws the dust from the dust extraction channel into the dust bucket through the air suction end. As a result, the clearance rate of the dust box of the sweeping robot is improved, thereby ensuring that the interior of the dust box of the sweeping robot is completely clean and preventing mildew. The maintenance station further includes a microprocessor electrically connected to the first fan, and the user can control the maintenance station through the settings in the microprocessor. The method includes at least:

  mating the sweeping robot with the maintenance station for charging;

  starting the second fan first, and then starting the first fan to extract dust from the sweeping robot; or, starting the first fan and the second fan at the same time; and

  when dust extraction is completed, turning off the first fan first, and then turning off the second fan; or, turning off the first fan and the second fan at the same time.

Specifically, a cleaning control method for a dust box of the present application includes methods shown in a first embodiment and a second embodiment below.

First embodiment

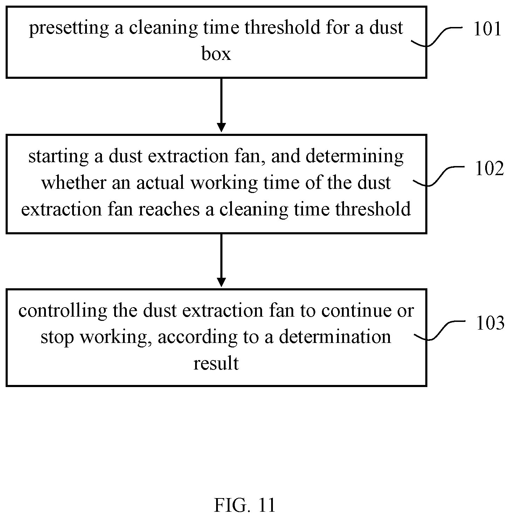

Referring to FIG. 11, in this embodiment, the cleaning control method for the dust box, includes:
step 101, presetting a cleaning time threshold of the dust box.

In one embodiment, the cleaning time threshold is a fixed dust extraction time. That is, after the fixed dust extraction time is set, when an actual working time of the first fan reaches the fixed dust extraction time, the first fan stops working. However, after this setting, if there is less dust and debris in the dust box, the first fan will still continue to work and run idly, thereby generating noise. Therefore, in another embodiment, before presetting the cleaning time threshold of the dust box, a capacity of the dust box and a working flow of the first fan can be obtained first. Then, according to the capacity of the dust box and the working flow of the first fan, an ideal cleaning time of the dust box is calculated.

Specifically, the ideal cleaning time is:

$$T\_min=A/B;$$

where A is the capacity of the dust box, and B is the working flow of the first fan.

The purpose of this setting is to determine whether there is dust and debris in the dust box by detecting the working flow of the first fan, and then to determine whether to make the first fan continue to work. Correspondingly, a flow sensor is arranged in the air channel. The flow sensor is used to detect the working flow of the first fan. The flow sensor is in signal connection with the microprocessor. In this embodiment, the type of the flow sensor is not specifically limited, as long as it can achieve the purpose of detecting the working flow of the first fan.

Then, a clean-up working time of the first fan is set, and the clean-up working time is longer than the ideal cleaning time. The clean-up working time is set as a cleaning time threshold of the dust box. Wherein, the clean-up working time is N times of the ideal cleaning time, where N is a positive integer, which is set according to the actual situation, and is not specifically limited here. N can be 1, or can be set as a threshold, which can be set by the user according to actual needs.

step 102: starting the second fan first, then starting the first fan; or starting the first fan and the second fan at the same time; and determining whether the actual working time of the first fan reaches the cleaning time threshold.

step 103: controlling the first fan and the second fan to continue or stop working according to a determination result; if the actual working time is greater than or equal to the cleaning time threshold, the microprocessor controls the first fan and the second fan to stop working sequentially or simultaneously; otherwise, the first fan and the second fan are controlled to continue to work.

In summary, by setting the cleaning time threshold of the dust box, when the actual working time of the first fan reaches the cleaning time threshold, the first fan is turned off, which is convenient and intelligent.

By obtaining the capacity of the dust box and the working flow of the first fan, the ideal cleaning time of the first fan is calculated according to the ratio of the two. According to the ideal cleaning time, the clean-up working time of the first fan is set, which is fast and can avoid the idling of the first fan, thereby improving user experience.

Second embodiment

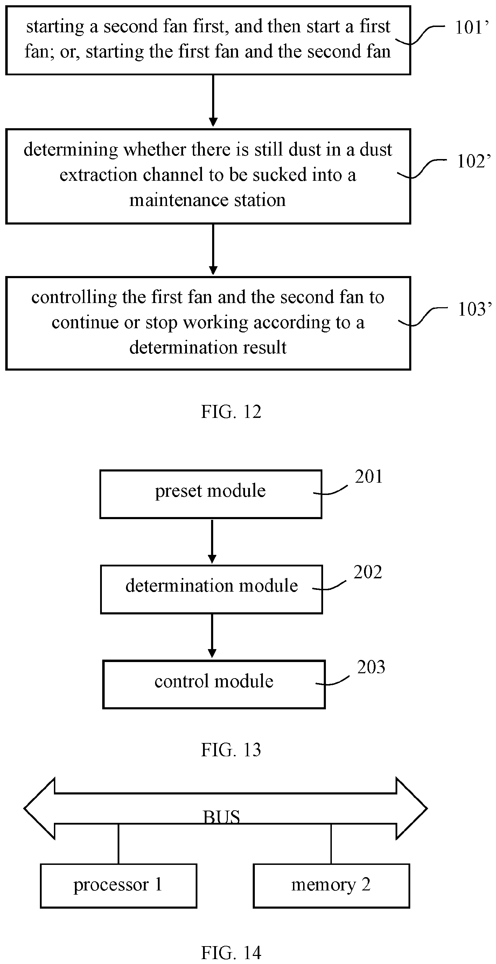

Referring to FIG. 12, in this embodiment, the cleaning control method for the dust box, includes:

    step 101', starting the second fan first, and then start the first fan; or, starting the first fan and the second fan at the same time;

    step 102', determining whether there is still dust in the dust extraction channel to be sucked into the maintenance station; and

    step 103', controlling the first fan and the second fan to continue or stop working according to a determination result; if no dust is sucked in, the microprocessor controls the first fan and the second fan to stop working sequentially or simultaneously; otherwise, the first fan and the second fan are controlled to continue to work.

In this embodiment, a dust detection device is provided in the dust extraction channel. Preferably, it is a dust sensor which is electrically connected to the microprocessor. The sensor is used to determine whether there is still dust drawn into the dust extraction channel, and then the microprocessor receives a sensor signal and controls the working state of the first fan. When the sensor determines that no dust is drawn into the dust extraction channel, the first fan is turned off to avoid idling, avoid wasting energy and avoid generating noise.

In the first embodiment and the second embodiment, the sweeping robot can control the working condition of the second fan through the processor disposed on it, and can also control the working condition of the second fan by the microprocessor of the maintenance station. Preferably, when the sweeping robot is mated with the maintenance station, the microprocessor is electrically connected to the second fan.

Third embodiment

FIG. 13 is a cleaning control device for a dust box of a sweeping robot provided by an embodiment of the present application, and the device includes at least:

    a preset module 201, configured to preset a cleaning time threshold of the dust box;

    a determination module 202, configured to determine whether there is still dust drawn into the maintenance station; or configured to determine whether an actual working time of the first fan has reached the cleaning time threshold after starting the first fan; and

    a control module 203, configured to control the first fan to continue or stop working according to a determination result.

For relevant details, refer to the above method embodiments.

It should be noted that: when the cleaning control device for the dust box of the sweeping robot provided in the above embodiments cleans the dust box, only the division of the above functional modules is used as an example for illustration. In practical applications, the above-mentioned functions can be assigned to different function modules as required. That is, the internal structure of the cleaning control device for the dust box of the sweeping robot is divided into different functional modules to complete all or part of the functions described above. In addition, the cleaning control device for the dust box of the sweeping robot provided by the above embodiment and the cleaning control method for the dust box of the sweeping robot belong to the same concept. The specific implementation process of the cleaning control device is detailed in the method embodiment, which will not be repeated here.

Fourth embodiment

FIG. 14 is a cleaning control device for a dust box of a sweeping robot provided by an embodiment of the present application. The device includes at least a processor 1 and a memory 2.

The processor 1 may include one or more processing cores, such as a 4-core processor, an 8-core processor, and the like. The processor 1 may be implemented in at least one hardware form among DSP (Digital Signal Processing), FPGA (Field-Programmable Gate Array), and PLA (Programmable Logic Array). The processor 1 may also include a main processor and a co-processor. The main processor is a processor for processing data in a wake-up state, and is also called a CPU (Central Processing Unit). The co-processor is a low-power processor for processing data in a standby state.

The memory 2 may include one or more computer-readable storage medium. The computer-readable storage medium may be non-transitory. The memory 2 may also include highspeed random access memory, as well as non-volatile memory, such as one or more disk storage devices, flash storage devices. In some embodiments, a non-transitory computer-readable storage medium in the memory 2 is used to store at least one instruction. The at least one instruction is loaded and executed by the processor 1 to implement the cleaning control method for the dust box of the sweeping robot provided in the method embodiments of the present application.

In some embodiments, the cleaning control device for the dust box of the sweeping robot may optionally further include: a peripheral device port and at least one peripheral device. The processor 1, the memory 2 and the peripheral device port can be connected through a BUS or a signal line. Each peripheral device can be connected to the peripheral device port through the BUS, the signal line or a circuit board. Illustratively, the peripheral devices include, but are not limited to, radio frequency circuits, touch screens, audio circuits, and power supplies etc.

Certainly, the cleaning control device for the dust box of the sweeping robot may further include fewer or more components, which is not limited in this embodiment.

Optionally, the present application provides a computer-readable storage medium. The computer-readable storage medium includes a program stored therein. The program is executed by the processor 1 to implement the cleaning control method for the dust box of the sweeping robot as described above.

Optionally, the present application also provides a computer product. The computer product includes the computer-readable storage medium. The program is stored in the computer-readable storage medium. The program is loaded and executed by the processor 1 to implement the cleaning control method for the dust box of the sweeping robot according to the above method embodiments.

Fifth embodiment

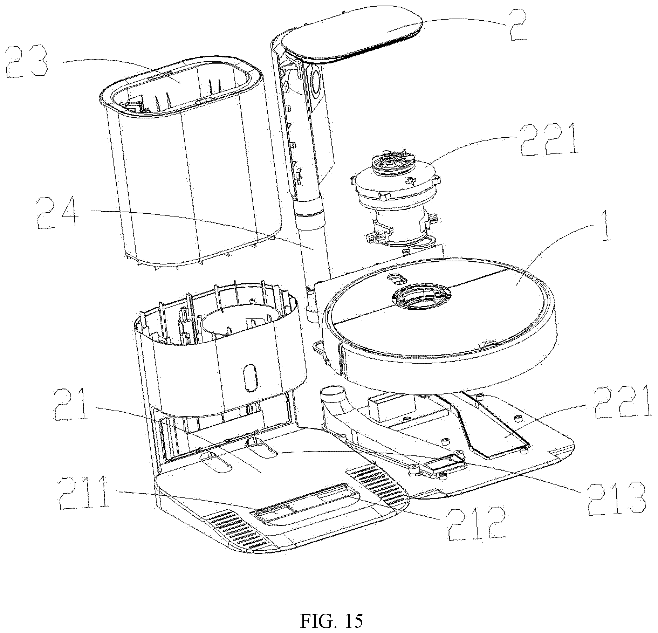

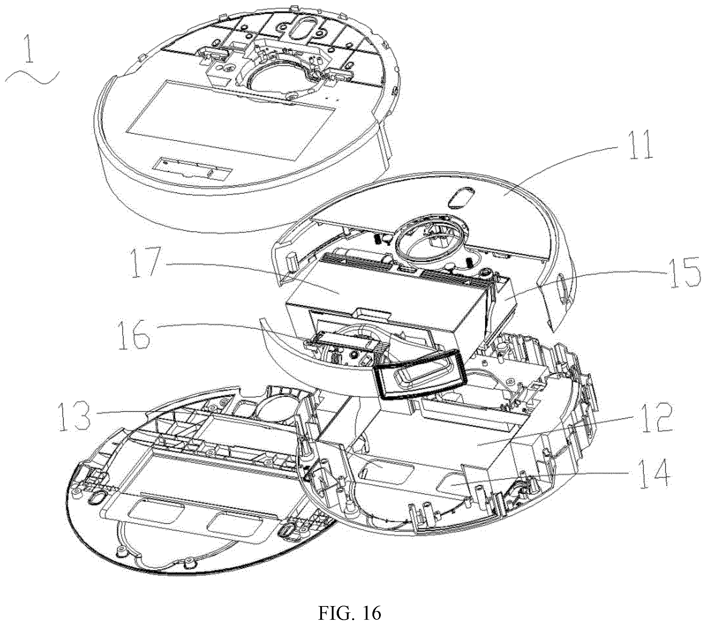

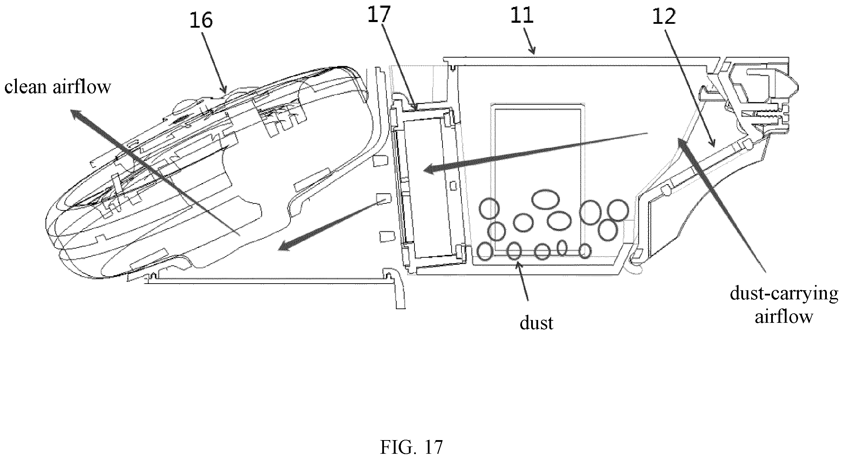

Referring to FIG. 15 to FIG. 17, an intelligent cleaning system shown in a preferred embodiment of the present application includes a sweeping robot 1 and a maintenance station 2 configured to mate with the sweeping robot 1. The maintenance station 2 in this embodiment can be used to perform functions such as charging and dust extraction for the sweeping robot 1.

The maintenance station 2 includes a first shell 21, a first fan 22 disposed in the first shell 21, and a charging assembly. The first shell 21 is provided with a dust extraction port 211 and an air discharge port 212. An air discharge end and an air suction end of the first fan 22 communicate with the air discharge port 212 and the dust extraction port 211, respectively. In this embodiment, in order to reduce the loss of air volume, the air discharge end of the first fan 22 and the air discharge port 212 are communicated with each other through an inclined channel 221. Similarly, the air suction end and the dust extraction port 211 can also be communicated with each other through an inclined channel. Indeed, in other embodiments, an arc-shaped air channel may be an arc-shaped channel or the like.

In this embodiment, the charging assembly and the sweeping robot 1 generally use a charging elastic sheet 213 for contact charging. The charging elastic sheet 213 and the first fan 22 are both in the prior art, which will not be described in detail here.

The sweeping robot 1 includes a second shell 11 and a working assembly disposed in the second shell 11. The second shell 11 is provided with a dust suction port 12, a dust outlet port 13 and an air blowing port 14. The dust suction port 12 is a conventional structure of the sweeping robot 1, and is usually provided at the bottom of the second shell 11. In this embodiment, the dust outlet port 13 and the air blowing port 14 are disposed at opposite ends of the second shell 11. The dust outlet port 13 is configured to mate with the dust extraction port 211. The air blowing port 14 is configured to mate with the air discharge port 212. The first fan 22 discharges the air from the air discharge port 212 and enters the second shell 11 from the air blowing port 14, which applies a certain kinetic energy to the dust in the second shell 11 to make the dust float up, and then the first fan 22 is used to suck the dust from the dust outlet port into the maintenance station 2. As a result, the clearance rate of the dust box of the sweeping robot 1 is improved. In addition, the working assembly is also provided in the second shell 11, so that the sweeping robot 1 can move and clean.

Specifically, the second shell 11 has a bottom surface and an accommodating space 110. The dust outlet port 13 and the air blowing port 14 are arranged at opposite ends of the bottom surface, respectively. More preferably, the bottom surface includes a first bottom surface 111 and a second bottom surface 112. In a vertical direction of the sweeping robot 1, a height of the second bottom surface 112 is higher than that of the first bottom surface 111, and the dust outlet port 13 and the air blowing port 14 are arranged on the first bottom surface 111. The dust suction port 12 is arranged on the second bottom surface 112. This arrangement ensures that the dust sucked into the second shell 11 will not easily fall out from the dust suction port 12. Meanwhile, the sweeping robot 1 of this embodiment is provided with an air inlet valve at the air blowing port 14, and a dust discharge valve is provided at the dust outlet port 13. When the sweeping robot 1 is in a working state, the accommodating space 110 is in a negative pressure state, the air inlet valve closes the air blowing port 14, the dust discharge valve closes the dust outlet port 13, and dust is sucked from the dust suction port 12. When the sweeping robot 1 is mated with the maintenance station 2, the air inlet valve opens the air blowing port 14, and the dust discharge valve opens the dust outlet port 13. As a result, the air blowing port 14 and the dust outlet port 13 communicate with the air discharge port 212 and the dust extraction port 211, respectively, so that the dust extraction work is performed.

In this embodiment, the working assembly includes a ground brush assembly 15 disposed at the dust suction port 12, a driving assembly for driving the ground brush assembly 15 to rotate, a moving assembly for moving the sweeping robot 1, and a second fan 16 for keeping the accommodating space 110 in a negative pressure state. The working assembly further includes a filter 17 which is disposed between the second fan 16 and the accommodating space 110. When the sweeping robot 1 is in the working state, the second fan 16 starts to work, so that the accommodating space 110 is in the negative pressure state. Under the action of the ground brush assembly 15 and the second fan 16, the external dust-carrying airflow enters the accommodating space 110 from the dust suction port 12. After passing through the filter 17, the dust stays in the accommodating space 110, and the airflow enters the second fan 16.

Of course, in order to realize the sealing of the accommodating space 110 , the second shell 11 is also provided with an end cover, and a sealing structure, etc., which are in the prior art and will not be described here.

In the intelligent cleaning system of the present application, the maintenance station 2 is further provided with a dust storage box 23 for storing the dust extracted from the second shell 11, and a dust extraction channel 24 communicating with the dust storage box 23 and the dust extraction port 211. A dust detection device is disposed in the dust extraction channel 24. When the charging elastic sheet on the sweeping robot is in contact with the charging elastic sheet 213 of the maintenance station 2, the first fan 22 of the maintenance station 2 starts to work so as to perform a dust extraction action. The dust in the dust box of the sweeping robot enters the dust storage box 23 through the dust extraction channel 24. When the dust detection device detects that no dust enters the dust extraction passage 24, the first fan 22 of the maintenance station 2 stops working. In this embodiment, the dust detection device includes a sensor and a microprocessor. The sensor is electrically connected to the microprocessor. The microprocessor is electrically connected to the first fan 22. The sensor is used to determine whether there is still dust drawn into the dust extraction channel 24, and then the microprocessor receives a sensor signal and controls the on or off of the first fan 22. When the sensor determines that no dust is drawn into the dust extraction channel 24, the first fan 22 is turned off to avoid idling, avoid wasting energy and avoid generating noise.

In this embodiment, an ultraviolet germicidal lamp is also provided in the dust storage box 23. Certainly, in other embodiments, the ultraviolet germicidal lamp may also be disposed in the dust extraction channel 24. However, disposing the ultraviolet germicidal lamp in the dust storage box 23 can ensure complete sterilization. When the charging elastic sheet of the sweeping robot is in contact with the charging elastic sheet 213 of the maintenance station 2, the first fan 22 of the maintenance station 2 starts to work so as to perform the dust extraction action. At this time, the dust storage box 23 and/or the ultraviolet germicidal lamp in the dust extraction channel 24 lights up. The dust enters the dust box of the maintenance station 2 through the dust extraction channel 24. This design can prevent a large amount of bacteria from being generated in the dust storage box 23 of the maintenance station 2, thereby avoiding secondary pollution to the air.

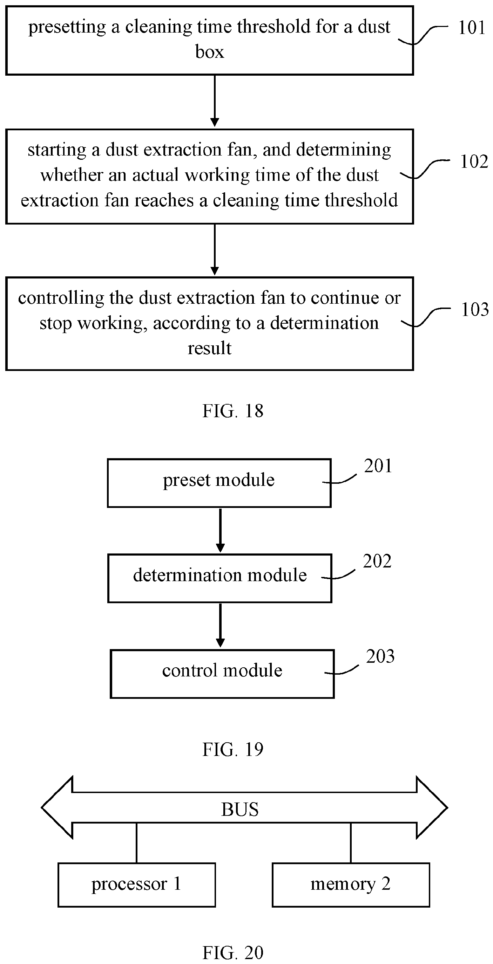

FIG. 18 is a cleaning control method for a dust box of a sweeping robot provided by an embodiment of the present application, which is applicable to a sweeping machine system. The sweeping machine system includes a sweeping robot and a dust-collecting bucket. The sweeping robot can be integrated with the dust-collecting bucket or set separately, depending on the actual situation, which is not specifically limited here. In this embodiment, the sweeping robot and the dust-collecting bucket are arranged separately. When the dust box of the sweeping robot is full of dust and debris, the sweeping robot mates with the dust-collecting bucket so as to clean the dust box.

The dust-collecting bucket includes a shell, a dust bucket arranged in the shell, an air channel for connecting the dust box and the dust bucket, and a dust extraction fan connected with the air channel. The dust extraction fan works to suck the dust and debris in the dust box into the dust bucket through the air channel, which is convenient and quick. The dust-collecting bucket also includes a microprocessor electrically connected with the dust extraction fan. The user can control the dust bucket through the settings in the microprocessor. The method includes at least:

step 101, presetting a cleaning time threshold required for cleaning the dust box.

In one embodiment, the cleaning time threshold is a fixed dust extraction time. That is, after the fixed dust extraction time is set, when an actual working time of the dust extraction fan reaches the fixed dust extraction time, the dust extraction fan stops working. However, after this setting, if there is less dust and debris in the dust box, the dust extraction fan will still continue to work and run idly, thereby generating noise. Therefore, in another embodiment, before presetting the cleaning time threshold of the dust box, a capacity of the dust box and a working flow of the dust extraction fan can be obtained first. Then, according to the capacity of the dust box, and an average working flow of the dust extraction fan obtained according to the capacity of the dust box, the ideal cleaning time of the dust box is calculated. Among them, the average working flow of the dust extraction fan is set according to the capacity of the dust box, or obtained by a sensor, which is not specifically limited here, and is determined according to the actual situation.

Specifically, the ideal cleaning time is:

$$T\_min=k*A/B;$$

where k >=1 and is a natural number, A is the capacity of the dust box, and B is the average working flow of the dust extraction fan.

The purpose of this setting is to determine whether there is dust and debris in the dust box by detecting the working flow of the dust extraction fan, and then to determine whether to make the dust extraction fan continue to work. As mentioned above, in this embodiment, a sensor is provided in the air channel. The sensor is used to detect the average working flow of the dust extraction fan. The sensor is in signal connection with the microprocessor. In this embodiment, the type of the sensor is not specifically limited, as long as it can achieve the purpose of detecting the working flow of the dust extraction fan.

Then, a clean-up working time of the dust extraction fan is set, and the clean-up working time is longer than the ideal cleaning time. The clean-up working time is set as a cleaning time threshold of the dust box. Wherein, the clean-up working time is N times of the ideal cleaning time, where N is a positive integer, which is set according to the actual situation, and is not specifically limited here. N can be 1, or can be set as a threshold, which can be set by the user according to actual needs.

step 102: starting the dust extraction fan, and determining whether the actual working time of the dust extraction fan reaches the cleaning time threshold.

step 103: controlling the dust extraction fan to continue or stop working according to a determination result; if the actual working time is greater than or equal to the cleaning time threshold, the microprocessor controls the dust extraction fan to stop working; otherwise, it controls the dust extraction fan to continue to work.

In summary, by setting the cleaning time threshold of the dust box, when the actual working time of the dust extraction fan reaches the cleaning time threshold, the dust extraction fan will be turned off, which is convenient and intelligent.

By obtaining the capacity of the dust box and the working flow of the dust extraction fan, the ideal cleaning time of the dust extraction fan is calculated according to the ratio of the two. According to the ideal cleaning time, the clean-up working time of the dust extraction fan is set, which is fast and can avoid the idling of the dust extraction fan, thereby improving user experience.

FIG. 19 is a cleaning control device for a dust box of a sweeping robot provided by an embodiment of the present application. The device includes at least:

a preset module 201, configured to preset a cleaning time threshold of the dust box;

a determination module 202, configured to determine whether an actual working time of a dust extraction fan reaches the cleaning time threshold after the dust extraction fan is started; and

a control module 203, configured to control the dust extraction fan to continue or stop working according to a determination result.

For relevant details, refer to the above method embodiments.

It should be noted that: when the cleaning control device for the dust box of the sweeping robot provided in the above embodiments cleans the dust box, only the division of the above functional modules is used as an example for illustration. In practical applications, the above-mentioned functions can be assigned to different function modules as required. That is, the internal structure of the cleaning control device for the dust box of the sweeping robot is divided into different functional modules to complete all or part of the functions described above. In addition, the cleaning control device for the dust box of the sweeping robot provided by the above embodiment and the cleaning control method for the dust box of the sweeping robot belong to the same concept. The specific implementation process of the cleaning control device is detailed in the method embodiment, which will not be repeated here.

FIG. 20 is a cleaning control device for a dust box of a sweeping robot provided by an embodiment of the present application. The device includes at least a processor 1 and a memory 2.

The processor 1 may include one or more processing cores, such as a 4-core processor, an 8-core processor, and the like. The processor 1 may be implemented in at least one hardware form among DSP (Digital Signal Processing), FPGA (Field-Programmable Gate Array), and PLA (Programmable Logic Array). The processor 1 may also include a main processor and a co-processor. The main processor is a processor for processing data in a wake-up state, and is also called a CPU (Central Processing Unit). The co-processor is a low-power processor for processing data in a standby state.

The memory 2 may include one or more computer-readable storage medium. The computer-readable storage medium may be non-transitory. The memory 2 may also include highspeed random access memory, as well as non-volatile memory, such as one or more disk storage devices, flash storage devices. In some embodiments, a non-transitory computer-readable storage medium in the memory 2 is used to store at least one instruction. The at least one instruction is loaded and executed by the processor 1 to implement the cleaning control method for the dust box of the sweeping robot provided in the method embodiments of the present application.

In some embodiments, the cleaning control device for the dust box of the sweeping robot may optionally further include: a peripheral device port and at least one peripheral device. The processor 1, the memory 2 and the peripheral device port can be connected through a BUS or a signal line. Each peripheral device can be connected to the peripheral device port through the BUS, the signal line or a circuit board. Illustratively, the peripheral devices include, but are not limited to, radio frequency circuits, touch screens, audio circuits, and power supplies etc.

Certainly, the cleaning control device for the dust box of the sweeping robot may further include fewer or more components, which is not limited in this embodiment.

Optionally, the present application provides a computer-readable storage medium. The computer-readable storage medium includes a program stored therein. The program is executed by the processor 1 to implement the cleaning control method for the dust box of the sweeping robot as described above.

Optionally, the present application also provides a computer product. The computer product includes the computer-readable storage medium. The program is stored in the computer-readable storage medium. The program is loaded and executed by the processor 1 to implement the cleaning control method for the dust box of the sweeping robot according to the above method embodiments.

The technical features of the above-described embodiments can be combined arbitrarily. In order to simplify the description, all possible combinations of the technical features in the above embodiments are not described. However, as long as there is no contradiction in the combination of these technical features, they should be considered to be within the scope of the description in this specification.

The above-mentioned embodiments only represent several embodiments of the present application, and the descriptions thereof are relatively specific and detailed, but should not be construed as a limitation on the scope of the patent application. It should be pointed out that for those skilled in the art, without departing from the concept of the present application, several modifications and improvements can be made, which all belong to the protection scope of the present application. Therefore, the protection scope of the present application shall be subject to the appended claims.

## Claims

1. An intelligent cleaning system, comprising: a sweeping robot and a maintenance station configured to mate with the sweeping robot, the maintenance station comprising:

   a first shell being provided with a dust extraction port and an air discharge port;
   a first fan arranged on the first shell, an air discharge end and an air suction end of the first fan communicating with the air discharge port and the dust extraction port, respectively; and
   a charging assembly disposed on the first shell and configured to charge the sweeping robot;
   the sweeping robot comprising:

   a second shell provided with a dust suction port, a dust outlet port and an air blowing port; the dust outlet port and the air blowing port are arranged at opposite ends of the second shell; the dust outlet port is configured to mate with the dust extraction port, and the air blowing port is configured to mate with the air discharge port; and
   a working assembly arranged on the second shell to drive the sweeping robot to move and clean.

2. The intelligent cleaning system according to claim 1, wherein the second shell has a bottom surface; the dust outlet port and the air flowing port are arranged at opposite ends of the bottom surface, respectively.

3. The intelligent cleaning system according to claim 2, wherein the bottom surface comprises a first bottom surface and a second bottom surface; a height of the second bottom surface is higher than that of the first bottom surface in a vertical direction of the sweeping robot; the dust outlet port and the air flowing port are arranged on the first bottom surface, and the dust suction port is arranged on the second bottom surface.

4. The intelligent cleaning system according to claim 1, wherein an air inlet valve is provided at the air flowing port; when the sweeping robot is in a working state, the air inlet valve closes the air flowing port; when the sweeping robot is mated with the maintenance station, the air inlet valve opens the air flowing port, so that the air flowing port communicates with the air discharge port.

5. The intelligent cleaning system according to claim 4, wherein a dust discharge valve is provided at the dust outlet port; when the sweeping robot is in the working state, the dust discharge valve closes the dust outlet port; when the sweeping robot is mated with the maintenance station, the dust discharge valve opens the dust outlet port, so that the dust outlet port communicates with the dust extraction port.

6. The intelligent cleaning system according to claim 1, wherein the second shell has an accommodating space; and when the sweeping robot is in a working state, the accommodating space is in a negative pressure state.

7. The intelligent cleaning system according to claim 6, wherein the working assembly comprises a ground brush assembly disposed at the dust suction port, a driving assembly driving the ground brush assembly to rotate, a moving assembly for moving the sweeping robot, and a second fan for making the accommodating space in a negative pressure state.

8. The intelligent cleaning system according to claim 7, wherein the working assembly further comprises a filter which is arranged between the second fan and the accommodating space; when the sweeping robot is in the working state, dust-carrying airflow enters the accommodating space from the dust suction port, and enters the second fan after passing through the filter.

9. The intelligent cleaning system according to claim 1, wherein the maintenance station further comprises a dust storage box for storing dust extracted from the second shell, and a dust extraction channel communicating with the dust storage box and the dust extraction port.

10. The intelligent cleaning system according to claim 1, wherein the air discharge end of the first fan communicates with the air discharge port through an arc-shaped air channel or an inclined air channel.

11. An intelligent cleaning system, comprising: a sweeping robot and a maintenance station configured to mate with the sweeping robot, the maintenance station comprising:

a first shell being provided with a dust extraction port;
a first fan arranged on the first shell, an air suction end of the first fan communicating with the dust extraction port so as to be used for extracting dust from the sweeping robot;
a cavity provided in the first shell, the cavity communicating with the dust extraction port through a dust extraction channel, a sterilization device being provided in the dust extraction channel and/or the cavity; and
a charging assembly disposed on the first shell and configured to charge the sweeping robot;
the sweeping robot comprising:

a second shell provided with a dust suction port and a dust outlet port, the dust outlet port being configured to mate with the dust extraction port; and
a working assembly arranged on the second shell to drive the sweeping robot to move and clean.

12. The intelligent cleaning system according to claim 11, wherein the first shell is further provided with an air discharge port, and an air discharge end of the first fan communicates with the air discharge port; and wherein the second shell is provided with an air flowing port which is mated with the air discharge port.

13. The intelligent cleaning system according to claim 12, wherein the second shell has a bottom surface; the dust outlet port and the air flowing port are arranged at opposite ends of the bottom surface, respectively.

14. The intelligent cleaning system according to claim 13, wherein the bottom surface comprises a first bottom surface and a second bottom surface; a height of the second bottom surface is higher than that of the first bottom surface in a vertical direction of the sweeping robot; the dust outlet port and the air flowing port are arranged on the first bottom surface, and the dust suction port is arranged on the second bottom surface.

15. The intelligent cleaning system according to claim 12, wherein an air inlet valve is provided at the air flowing port; when the sweeping robot is in a working state, the air inlet valve closes the air flowing port; when the sweeping robot is mated with the maintenance station, the air inlet valve opens the air flowing port, so that the air flowing port communicates with the air discharge port.

16. The intelligent cleaning system according to claim 15, wherein a dust discharge valve is provided at the dust outlet port; when the sweeping robot is in the working state, the dust discharge valve closes the dust outlet port; when the sweeping robot is mated with the maintenance station, the dust discharge valve opens the dust outlet port, so that the dust outlet port communicates with the dust extraction port.

17. The intelligent cleaning system according to claim 11, wherein the second shell has an accommodating space; and when the sweeping robot is in a working state, the accommodating space is in a negative pressure state.

18. The intelligent cleaning system according to claim 17, wherein the working assembly comprises a ground brush assembly disposed at the dust suction port, a driving assembly driving the ground brush assembly to rotate, a moving assembly for moving the sweeping robot, and a second fan for making the accommodating space in a negative pressure state.

19. The intelligent cleaning system according to claim 18, wherein the working assembly further comprises a filter which is arranged between the second fan and the accommodating space; when the sweeping robot is in the working state, dust-carrying airflow enters the accommodating space from the dust suction port, and enters the second fan after passing through the filter.

20. The intelligent cleaning system according to claim 11, wherein a dust detection device is provided in the dust extraction channel, the dust detection device comprises a sensor and a microprocessor, the sensor is electrically connected to the microprocessor, and the microprocessor is electrically connected to the first fan.

21. A dust-full detection system for a dust-collecting bucket, comprising:

an acquisition unit comprising a sensor and/or a detection circuit, the sensor and/or the detection circuit being configured to detect a parameter of the dust-collecting bucket; a processing unit configured to receive the parameter and determine whether the parameter exceeds a preset threshold; and an alarm unit configured to remind a user to perform a related operation; wherein the processing unit instructs the alarm unit to send out indication information according to a determination result.

22. The dust-full detection system for the dust-collecting bucket according to claim 21, wherein when the dust-collecting bucket is working, the parameter is a parameter of a current and/or a parameter of a rotation speed of a dust extraction motor of the dust-collecting bucket.

23. The dust-full detection system for the dust-collecting bucket according to claim 21, wherein when the dust-collecting bucket is working, the parameter is a parameter of an air pressure in a dust bucket of the dust-collecting bucket.

24. The dust-full detection system for the dust-collecting bucket according to claim 21, wherein when the dust-collecting bucket is working, the parameter is a parameter of a weight of a dust bucket of the dust-collecting bucket.

25. The dust-full detection system for the dust-collecting bucket according to claim 21, wherein when the parameter exceeds the preset threshold, the alarm unit alarms to remind the user to clean the dust-collecting bucket.

26. The dust-full detection system for the dust-collecting bucket according to claim 21, wherein when the dust-collecting bucket is working, the parameter is a parameter of in-position time of a dust bucket of the dust-collecting bucket.

27. The dust-full detection system for the dust-collecting bucket according to claim 21, wherein when the dust-collecting bucket is working, the parameter is a parameter of working times of a dust extraction motor of the dust-collecting bucket.

28. The dust-full detection system for the dust-collecting bucket according to claim 21, wherein when the dust-collecting bucket is in operation, the parameter is a parameter of working times of a dust extraction motor within an in-position time of a dust bucket of the dust-collecting bucket.

29. A dust-full detection method for a dust-collecting bucket, comprising:

detecting a parameter of the dust-collecting bucket; determine whether the parameter exceeds a preset threshold; and sending indication information according to a determination result, and the indication information being alarm information.

30. The dust-full detection method for the dust-collecting bucket as claimed in claim 29, further comprising: sending an indication message to remind a user to clean the dust-collecting bucket when the parameter exceeds the preset threshold.

31. A cleaning control method for a dust box of a sweeping robot, the sweeping robot configured to mate with a maintenance station, the maintenance station having a first fan, the sweeping robot having a second fan, the method comprising:

mating the sweeping robot with the maintenance station for charging;
starting the second fan first, and then starting the first fan to extract dust from the sweeping robot; or, starting the first fan and the second fan at the same time; and
when dust extraction is completed, turning off the first fan first, and then turning off the second fan; or, turning off the first fan and the second fan at the same time.

32. The cleaning control method for the dust box of the sweeping robot according to claim 31, further comprising:
determining whether there is still dust drawn into the maintenance station; if not, turning off the first fan, and the dust extraction is completed; if yes, continuing the dust extraction.

33. The cleaning control method for the dust box of the sweeping robot according to claim 32, wherein an air discharge end of the first fan communicates with the sweeping robot through an air channel, an air suction end of the first fan communicates with the sweeping robot through a dust extraction channel; a dust detection device is arranged in the dust extraction channel to detect whether there is still dust drawn into the dust extraction channel.

34. The cleaning control method for the dust box of the sweeping robot according to claim 31, further comprising:
before performing the dust extraction, presetting a cleaning time threshold of the dust box; determining whether an actual working time of the first fan reaches the cleaning time threshold; and controlling the first fan to continue or stop working according to a determination result.

35. The cleaning control method for the dust box of the sweeping robot according to claim 34, further comprising:

before presetting the cleaning time threshold of the dust box, obtaining a capacity of the dust box and a working flow of the first fan; and calculating an ideal cleaning time of the dust box according to the capacity of the dust box and the working flow of the first fan; wherein the ideal cleaning time is:

$$T\_min = A/B;$$

where A is the capacity of the dust box, and B is the working flow of the first fan.

36. The cleaning control method for the dust box of the sweeping robot according to claim 35, further comprising:

setting a clean-up working time of the first fan, the clean-up working time being longer than the ideal cleaning time; and
setting the clean-up working time as the cleaning time threshold of the dust box; wherein the clean-up working time is N times of the ideal cleaning time, and N is a positive integer.

37. The cleaning control method for the dust box of the sweeping robot according to claim 36, wherein the first fan is connected with the dust box through an air channel, a flow sensor is disposed in the air channel, and the flow sensor is configured to detect the working flow of the first fan.

38. A cleaning control device for a dust box of a sweeping robot, the sweeping robot configured to mate with a maintenance station, the maintenance station having a first fan, the sweeping robot having a second fan, the device comprising:

a preset module configured to preset a cleaning time threshold of the dust box;
a determination module configured to determine whether there is still dust drawn into the maintenance station; or configured to determine whether an actual working time of the first fan has reached the cleaning time threshold after starting the first fan; and
a control module configured to control the first fan according to a determination result; or, configured to control the first fan and the second fan to continue or stop working.

39. A cleaning control device for a dust box of a sweeping robot, the device comprising a processor and a memory, a program being stored in the memory, and the program being loaded and executed by the processor to implement the cleaning control method for the dust box of the sweeping robot according to any one of claims 31 to 37.

40. A computer-readable storage medium, comprising a program stored in the storage medium; the program being executed by a processor to implement the cleaning control method for the dust box of the sweeping robot according to any one of claims 31 to 37.

41. A cleaning control method for a dust box of a sweeping robot, the sweeping robot configured to mate with a dust-collecting bucket, the sweeping robot having a dust box, the dust-collecting bucket having a dust extraction fan, the method comprising:

> presetting a cleaning time threshold required for cleaning the dust box;
> starting the dust extraction fan, and determining whether an actual working time of the dust extraction fan reaches the cleaning time threshold; and
> controlling the dust extraction fan to continue or stop working, according to the determination result.

42. The cleaning control method for the dust box of the sweeping robot according to claim 41, further comprising:

> before presetting the cleaning time threshold required for cleaning the dust box, obtaining a capacity of the dust box, and obtaining an average working flow of the dust extraction fan according to the capacity of the dust box; and
> calculating an ideal cleaning time of the dust box according to the capacity of the dust box and the average working flow of the dust extraction fan; wherein the ideal cleaning time is:

$$T\_min = k*A/B;$$

> where k >=1 and is a natural number, A is the capacity of the dust box, and B is the average working flow of the dust extraction fan.

43. The cleaning control method for the dust box of the sweeping robot according to claim 42, further comprising:

> setting a clean-up working time of the dust extraction fan, and the clean-up working time being longer than the ideal cleaning time; and
> setting the clean-up working time as a cleaning time threshold required for cleaning the dust box.

44. The cleaning control method for the dust box of the sweeping robot according to claim 43, wherein the clean-up working time is N times the ideal cleaning time, where N is a positive integer.

45. The cleaning control method for a dust box of a sweeping robot according to claim 42, wherein the dust extraction fan is connected with the dust box through an air channel, and a sensor is disposed in the air channel;
the sensor is configured to detect the average working flow of the dust extraction fan.

46. The cleaning control method for the dust box of the sweeping robot according to claim 41, wherein the cleaning time threshold is a fixed dust extraction time.

47. A cleaning control device for a dust box of a sweeping robot, the device comprising:

> a preset module configured to preset a cleaning time threshold of the dust box;
> a determination module configured to determine whether an actual working time of a dust extraction fan reaches the cleaning time threshold after the dust extraction fan is started; and
> a control module configured to control the dust extraction fan to continue or stop working according to a determination result.

48. A cleaning control device for a dust box of a sweeping robot, the device comprising a processor and a memory, a program being stored in the memory, and the program being loaded and executed by the processor to implement the cleaning control method for the dust box of the sweeping robot according to any one of claims 41 to 46.

49. A computer-readable storage medium, comprising a program stored in the storage medium, the program being executed by a processor to implement the cleaning control method for the dust box of the sweeping robot according to any one of claims 41 to 46.

EP 4 212 084 A1

FIG. 1

EP 4 212 084 A1

FIG. 2

FIG. 3

FIG. 4

FIG. 5

clean airflow

dust-carrying airflow

dust

FIG. 6

EP 4 212 084 A1

acquisition unit → processing unit → alarm unit

FIG. 7

detecting a parameter of a dust-collecting bucket

↓

determining whether the parameter exceeds a preset threshold

↓

sending indication information according to a determination result, and the indication information being alarm information

FIG. 8

FIG. 9

mating a sweeping robot with a maintenance station

starting a second fan first, and then starting a first fan to extract dust from a dust box; or, starting the first fan and the second fan at the same time

when dust extraction is completed, turning off the first fan first, and then turning off the second fan; or, turning off the first fan and the second fan at the same time

FIG. 10

| presetting a cleaning time threshold for a dust box | 101 |

↓

| starting a dust extraction fan, and determining whether an actual working time of the dust extraction fan reaches a cleaning time threshold | 102 |

↓

| controlling the dust extraction fan to continue or stop working, according to a determination result | 103 |

FIG. 11

starting a second fan first, and then start a first fan; or, starting the first fan and the second fan — 101'

determining whether there is still dust in a dust extraction channel to be sucked into a maintenance station — 102'

controlling the first fan and the second fan to continue or stop working according to a determination result — 103'

FIG. 12

preset module — 201

determination module — 202

control module — 203

FIG. 13

BUS

processor 1     memory 2

FIG. 14

FIG. 15

EP 4 212 084 A1

FIG. 16

clean airflow

16   17   11   12

dust

dust-carrying
airflow

FIG. 17

EP 4 212 084 A1

presetting a cleaning time threshold for a dust box — 101

↓

starting a dust extraction fan, and determining whether an actual working time of the dust extraction fan reaches a cleaning time threshold — 102

↓

controlling the dust extraction fan to continue or stop working, according to a determination result — 103

FIG. 18

preset module — 201

↓

determination module — 202

↓

control module — 203

FIG. 19

BUS

processor 1          memory 2

FIG. 20

## INTERNATIONAL SEARCH REPORT

| International application No. |
| --- |
| **PCT/CN2021/115247** |

### A. CLASSIFICATION OF SUBJECT MATTER

A47L 11/24(2006.01)i; A47L 11/40(2006.01)i; A61L 2/10(2006.01)i; G01F 23/18(2006.01)i; G08B 21/18(2006.01)i; G05B 19/042(2006.01)i

According to International Patent Classification (IPC) or to both national classification and IPC

### B. FIELDS SEARCHED

Minimum documentation searched (classification system followed by classification symbols)

A47L A61L G01F G08B G05B

Documentation searched other than minimum documentation to the extent that such documents are included in the fields searched

Electronic data base consulted during the international search (name of data base and, where practicable, search terms used)

CNABS; CNTXT; VEN; WOTXT; EPTXT; USTXT; CNKI; IEEE: 扫地机器人, 维护站, 灰, 尘, 垃圾, 回收, 集尘, 尘盒, 悬浮, 排风, 吹风, 电机, 电流, 转速, 气压, 工作流量, 时间, 阈值, robot, maintenance station, dust, garbage, recycle, collect+, dust box+, suspend+, blow+, engine, current, rotate speed, pressure, time, work+ flow, threshold value

### C. DOCUMENTS CONSIDERED TO BE RELEVANT

| Category* | Citation of document, with indication, where appropriate, of the relevant passages | Relevant to claim No. |
| --- | --- | --- |
| PX | CN 112022007 A (ZHUICHUANG TECHNOLOGY (SUZHOU) CO., LTD.) 04 December 2020 (2020-12-04)<br>description, paragraphs [0045]-[0100], and figures 1-8 | 1-20, 31-40 |
| PX | CN 112013923 A (ZHUICHUANG TECHNOLOGY (SUZHOU) CO., LTD.) 01 December 2020 (2020-12-01)<br>claims 1-10 | 21-30 |
| PX | CN 112006613 A (ZHUICHUANG TECHNOLOGY (SUZHOU) CO., LTD.) 01 December 2020 (2020-12-01)<br>claims 1-9 | 41-49 |
| X | CN 102334943 A (SAMSUNG ELECTRONICS CO., LTD.) 01 February 2012 (2012-02-01)<br>description, paragraphs [0111]-[0192], and figures 1-10 | 1-30 |
| X | CN 107529930 A (IROBOT CORPORATION) 02 January 2018 (2018-01-02)<br>description, paragraphs [0070]-[0084], and figures 1-5 | 21-30, 38, 41, 46-49 |
| A | CN 111466831 A (BEIJING QIHOO TECHNOLOGY CO., LTD.) 31 July 2020 (2020-07-31)<br>entire document | 1-49 |

☐ Further documents are listed in the continuation of Box C.　　☑ See patent family annex.

\* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
| --- | --- |
| **23 October 2021** | **18 November 2021** |

| Name and mailing address of the ISA/CN | Authorized officer |
| --- | --- |
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

International application No.

**PCT/CN2021/115247**

| Box No. III | Observations where unity of invention is lacking (Continuation of item 3 of first sheet) |
|---|---|

This International Searching Authority found multiple inventions in this international application, as follows:

[1]    Claims 1 and 11 relate to an intelligent cleaning system;

[2]    Claim 21 relates to a barrel-full detection system of a dust collection barrel;

[3]    Claim 29 relates to a barrel-full detection method for a dust collection barrel;

[4]    Claims 31 and 41 relate to a dust bin cleaning control method for a cleaning robot;

[5]    Claims 38 and 47 relate to dust bin cleaning control device for a cleaning robot;

[6]    There is no same or corresponding special technical feature between claims 1 and 11 and claims 21, 29, 31, 38, 41, and 47, and between claims 21 and 29 and claims 31, 38, 41, and 47, said claims do not belong to a single general inventive concept, and therefore do not comply with the requirement of unity of invention as defined in PCT Rule 13.1.

1. ☐    As all required additional search fees were timely paid by the applicant, this international search report covers all searchable claims.

2. ☑    As all searchable claims could be searched without effort justifying additional fees, this Authority did not invite payment of additional fees.

3. ☐    As only some of the required additional search fees were timely paid by the applicant, this international search report covers only those claims for which fees were paid, specifically claims Nos.:

4. ☐    No required additional search fees were timely paid by the applicant. Consequently, this international search report is restricted to the invention first mentioned in the claims; it is covered by claims Nos.:

**Remark on Protest**    ☐    The additional search fees were accompanied by the applicant's protest and, where applicable, the payment of a protest fee.

☐    The additional search fees were accompanied by the applicant's protest but the applicable protest fee was not paid within the time limit specified in the invitation.

☐    No protest accompanied the payment of additional search fees.

Form PCT/ISA/210 (continuation of first sheet) (January 2015)

<div align="center">

**INTERNATIONAL SEARCH REPORT**
**Information on patent family members**

</div>

International application No.

**PCT/CN2021/115247**

| Patent document cited in search report | | | Publication date (day/month/year) | Patent family member(s) | | | Publication date (day/month/year) |
|---|---|---|---|---|---|---|---|
| CN | 112022007 | A | 04 December 2020 | CN | 213588175 | U | 02 July 2021 |
| CN | 112013923 | A | 01 December 2020 | | None | | |
| CN | 112006613 | A | 01 December 2020 | | None | | |
| CN | 102334943 | A | 01 February 2012 | US | 2018296051 | A1 | 18 October 2018 |
| | | | | CN | 102342805 | A | 08 February 2012 |
| | | | | CN | 106264335 | A | 04 January 2017 |
| | | | | US | 2012011677 | A1 | 19 January 2012 |
| | | | | US | 8756751 | B2 | 24 June 2014 |
| | | | | EP | 2517610 | A1 | 31 October 2012 |
| | | | | EP | 2517610 | B1 | 30 June 2021 |
| | | | | US | 2012013907 | A1 | 19 January 2012 |
| | | | | US | 9186030 | B2 | 17 November 2015 |
| | | | | KR | 20160091312 | A | 02 August 2016 |
| | | | | KR | 101680884 | B1 | 12 December 2016 |
| | | | | EP | 2407074 | A2 | 18 January 2012 |
| | | | | EP | 2407074 | A3 | 07 November 2012 |
| | | | | EP | 2407074 | B1 | 14 June 2017 |
| | | | | KR | 20120007943 | A | 25 January 2012 |
| | | | | KR | 101483541 | B1 | 19 January 2015 |
| | | | | KR | 20140119674 | A | 10 October 2014 |
| | | | | KR | 101648880 | B1 | 17 August 2016 |
| | | | | US | 2012011676 | A1 | 19 January 2012 |
| | | | | US | 9027199 | B2 | 12 May 2015 |
| | | | | US | 2016029865 | A1 | 04 February 2016 |
| | | | | US | 10028631 | B2 | 24 July 2018 |
| | | | | CN | 102334945 | A | 01 February 2012 |
| | | | | CN | 102334945 | B | 16 March 2016 |
| | | | | KR | 20160137494 | A | 30 November 2016 |
| | | | | CN | 102334943 | B | 01 March 2017 |
| | | | | CN | 102342805 | B | 01 March 2017 |
| | | | | DE | 202011110010 | U1 | 27 September 2012 |
| | | | | AT | 12830 | U2 | 15 October 2012 |
| CN | 107529930 | A | 02 January 2018 | EP | 3313255 | A1 | 02 May 2018 |
| | | | | EP | 3313255 | A4 | 20 February 2019 |
| | | | | EP | 3313255 | B1 | 17 June 2020 |
| | | | | ES | 2818116 | T3 | 09 April 2021 |
| | | | | AU | 2015400076 | A1 | 30 November 2017 |
| | | | | AU | 2015400076 | B2 | 27 August 2020 |
| | | | | WO | 2016209309 | A1 | 29 December 2016 |
| | | | | CN | 109431376 | A | 08 March 2019 |
| | | | | JP | 2021035519 | A | 04 March 2021 |
| | | | | JP | 2018522613 | A | 16 August 2018 |
| | | | | JP | 6786521 | B2 | 18 November 2020 |
| | | | | US | 9462920 | B1 | 11 October 2016 |
| | | | | EP | 3777629 | A1 | 17 February 2021 |
| | | | | CN | 109528088 | A | 29 March 2019 |
| | | | | AU | 2020277235 | A1 | 24 December 2020 |
| | | | | CN | 109431376 | B | 11 June 2021 |
| CN | 111466831 | A | 31 July 2020 | | None | | |

Form PCT/ISA/210 (patent family annex) (January 2015)